# EUROPEAN PATENT SPECIFICATION

(11) **EP 2 718 438 B1**
(45) Date of publication and mention of the grant of the patent: **12.08.2015**
(21) Application number: 12732785.6
(22) Date of filing: 08.06.2012
(51) Int. Cl.: C12N 15/113, C12N 9/04, C12N 9/12, A61P 35/00, A61P 37/00

(54) **MODULATORS OF ADP-DEPENDENT GLUCOKINASE (ADPGK) FOR THERAPY**
MODULATOREN VON ADP-ABHÄNGIGER GLUCOKINASE (ADPGK) FÜR DIE THERAPIE
MODULATEURS DE GLUCOKINASE ADP-DÉPENDANTE (ADPGK) À VISÉE THÉRAPEUTIQUE

(30) Priority: 09.06.2011 EP 11004729
(43) Date of publication of application: 16.04.2014
(73) Proprietor: Deutsches Krebsforschungszentrum, 69120 Heidelberg (DE)
(72) Inventor: KRAMMER, Peter, 69120 Heidelberg (DE); KAMINSKI, Marcin M., 69126 Heidelberg (DE); GÜLOW, Karsten, 69198 Schriesheim (DE); SAUER, Sven W., 68162 Mannheim (DE)
(74) Representative: Schüssler, Andrea
(86) International application number: PCT/EP2012/002444
(87) International publication number: WO 2012/167944

(56) References cited:
- EP-A1- 1 752 536
- WO-A1-99/23108
- WO-A2-2005/024603
- WO-A2-2008/138578
- US-A1- 2007 148 664
- US-A1- 2010 209 914
- RICHTER SUSAN ET AL: "Role of the novel glucose-phosphorylating enzyme ADP-dependent glucokinase in human cancer cell lines featuring knockouts generated by zinc finger nucleases", PROCEEDINGS OF THE AMERICAN ASSOCIATION FOR CANCER RESEARCH ANNUAL MEETING, vol. 51 April 2011 (2011-04), XP002661597, 101ST ANNUAL MEETING OF THE AMERICAN-ASSOCIATION-FOR-CANCER-RESEARCH; WASHINGTON, DC, USA; APRIL 17 -21, 2010 ISSN: 0197-016X Retrieved from the Internet: URL:http://www.abstractsonline.com/Plan/Vi ewAbstract.aspx?mID=2521&sKey=634ef647-ef7 2-48d9-848c-d9bae614edfe&cKey=055b8c59-7d2 5-4069-ab71-980366d788b8&mKey=%7b0591FA3B- AFEF-49D2-8E65-55F41EE8117E%7d [retrieved on 2011-10-18]
- H PELICANO ET AL: "Glycolysis inhibition for anticancer treatment", ONCOGENE, vol. 25, no. 34, 7 August 2006 (2006-08-07), pages 4633-4646, XP055009657, ISSN: 0950-9232, DOI: 10.1038/sj.onc.1209597
- SCATENA ROBERTO ET AL: "Glycolytic enzyme inhibitors in cancer treatment.", EXPERT OPINION ON INVESTIGATIONAL DRUGS, vol. 17, no. 10, October 2008 (2008-10), pages 1533-1545, XP002661595, ISSN: 1744-7658
- N. J. MACIVER ET AL: "Glucose metabolism in lymphocytes is a regulated process with significant effects on immune cell function and survival", JOURNAL OF LEUKOCYTE BIOLOGY, vol. 84, no. 4, 1 October 2008 (2008-10-01), pages 949-957, XP055009842, ISSN: 0741-5400, DOI: 10.1189/jlb.0108024
- KRAWCZYK CONNIE M ET AL: "Toll-like receptor-induced changes in glycolytic metabolism regulate dendritic cell activation", BLOOD, vol. 115, no. 23, June 2010 (2010-06), pages 4742-4749, XP002661596,
- HEATHER R. CHRISTOFK ET AL: "The M2 splice isoform of pyruvate kinase is important for cancer metabolism and tumour growth", NATURE, vol. 452, no. 7184, 13 March 2008 (2008-03-13), pages 230-233, XP055009852, ISSN: 0028-0836, DOI: 10.1038/nature06734
- CHOWDHURY S K R ET AL: "High activity of mitochondrial glycerophosphate dehydrogenase and glycerophosphate-dependent ROS production in prostate cancer cell lines", BIOCHEMICAL AND BIOPHYSICAL RESEARCH COMMUNICATIONS, vol. 333, no. 4, 12 August 2005 (2005-08-12), pages 1139-1145, XP004962294, ACADEMIC PRESS INC. ORLANDO, FL, US ISSN: 0006-291X, DOI: 10.1016/J.BBRC.2005.06.017
- CHOWDHURY SUBIR K ROY ET AL: "Increased expression of mitochondrial glycerophosphate dehydrogenase and antioxidant enzymes in prostate cancer cell lines/cancer.", FREE RADICAL RESEARCH, vol. 41, no. 10, October 2007 (2007-10), pages 1116-1124, XP008148616, ISSN: 1071-5762
- MEYNARD DELPHINE ET AL: "Functional analysis of the molecular determinants of the cytotoxicity of oxaliplatin in colorectal cancer cell lines.", PROCEEDINGS OF THE AMERICAN ASSOCIATION FOR CANCER RESEARCH ANNUAL MEETING, vol. 45 March 2004 (2004-03), XP002669316, & 95TH ANNUAL MEETING OF THE AMERICAN-ASSOCIATION-FOR-CANCER-RESEARCH; ORLANDO, FL, USA,MARCH 27 -31, 2004 ISSN: 0197-016X Retrieved from the Internet: URL:http://www.aacrmeetingabstracts.org/cg i/content/abstract/2004/1/518-c [retrieved on 2012-02-10]

## Description

The present invention provides a compound capable of modulating (a) the biological activity of ADP-dependent glucokinase (ADPGK) or (b) the expression of the gene encoding ADPGK for use in treating a neoplasm, an autoimmune disease or Graft-versus-Host-Disease (GvHD).

The inhibition of glycolysis in connection with the Warburg effect for cancer treatment has been descriebed by Pelicano, H. et al. (50).

Scatena, R. et al. describe inhibitors of glycolytic enzymes for the treatment of cancer in connection with the Warburg effect (51).

Some possible therapeutics strategies for the treatment of cancer and/or autoimmune diseases have been described by Maciver, N.J. et al. (52). Also in this reference a modified glucose metabolism, glycolysis and the Warburg effect are described.

Krawczyk, C. et al. describe changes of the glycolytic metabolism for the activation regulation of dendritic cells (53). It concerns Toll-Like-Receptor (TLR) agonists wherein in particular the protein AMP is discussed.

Despite enormous investments of financial and human resources, cancer remains one of the major causes of death. Many management options for cancer exist including: chemotherapy, radiation therapy, surgery, monoclonal antibody therapy and other methods. Which treatments are used depends upon the type of cancer, the location and grade of the tumor, and the stage of the disease, as well as the general state of a person's health. Complete removal of the cancer without damage to the rest of the body is the goal of treatment for most cancers. Sometimes this can be accomplished by surgery, but the propensity of cancers to invade adjacent tissue or to spread to distant sites by microscopic metastasis often limits its effectiveness. Surgery often required the removal of a wide surgical margin or a free margin. The width of the free margin depends on the type of the cancer, the method of removal (CCPDMA, Mohs surgery, POMA, etc.). The margin can be as little as 1 mm for basal cell cancer using CCPDMA or Mohs surgery, to several centimeters for aggressive cancers. The effectiveness of chemotherapy is often limited by toxicity to other tissues in the body. Radiation can also cause damage to normal tissue. Because cancer is a class of diseases, it is unlikely that there will ever be a single "cure for cancer" any more than there will be a single treatment for all infectious diseases. Angiogenesis inhibitors were once thought to have potential as a "silver bullet" treatment applicable to many types of cancer, but this has not been the case in practice.

Immunotherapy of cancer has also been described in the art for a number of approaches, including early attempts of cell-based cancer vaccines consisting of killed autologous tumor cells or tumor cell lysates mixed with adjuvants, such as *Bacillus* Calmette Guerin (BCG) and *Corynebacterium parvum,* in an attempt to amplify tumor-specific immune responses. Further immunotherapeutic strategies include gene therapy, administration of small molecular inhibitors and activators, anti-sense oligonucleotides, vaccines, activated autologous cells, antibodies, as well as cytokines and chemokines, natural or recombinant proteins, autologous cells modified in vitro.

In spite of the length of time that these therapies have been investigated, there remains a need for improved strategies for tumor therapy as well as therapy of immune diseases.

It is, thus, the object of the present invention to provide a safe and effective means for such therapies.

According to the invention this is achieved by the subject matters defined in the claims. Activation via the T cell receptor (TCR) drives T cells into rapid proliferation and differentiation. At the stage of PLCγ1 induction, the TCR response splits into two pathways. Inositol 3,4,5-triphosphate induces a rise in intracellular Ca²⁺ concentration and activation of Ca²⁺-dependent transcription factors, *e.g.* NF-AT. Diacylglycerol (DAG) activates PKCθ and RasGRP proteins leading to triggering of NF-κB and AP-1. These three transcription factors essentially control T cell activation-induced gene expression. Thus, simultaneous treatment with Ca²⁺ ionophore, *e.g*. ionomycin (Iono), and a DAG mimetic, phorbol 12-myristate 13-acetate (PMA), yields full T cell activation-driven transcriptional response. It has been shown that T cell activation is paralleled by transient generation of low, physiologically relevant levels of ROS, i.e. a H₂O₂-mediated oxidative signal, which facilitates activation of oxidation-dependent transcription factors, NF-κB and AP-1 (*1, 2*). The oxidative signal is indispensible for T cell activation. Together with Ca²⁺ influx, it constitutes a minimal requirement for T cell activation-induced gene expression (*e.g*. IL-2, IL-4, CD95L). Neither signal is sufficient by itself (*1, 3, 4*).

Several different enzymatic sources, such as the mitochondrial respiratory chain, lipooxygenases and NADPH oxidases, NOX2 and DUOX2, were described to participate in T cell activation-triggered ROS production (*1, 3, 5-8*). Previous work demonstrates a crucial role for mitochondria as the source of the oxidative signal. Using a variety of experimental approaches, it could be shown that TCR proximal signalosome-mediated PKCθ activation drives ROS production from mitochondrial respiratory complex I. In addition, generation of the oxidative signal was shown to be blocked by metformin, an anti-diabetic drug (3).

T cell activation-induced gene expression depends on glucose uptake (9, *10).* Furthermore, T cell activation is accompanied by a metabolic switch from mitochondrial ATP production to aerobic glycolysis, *i.e.* the Warburg effect (*11-13*), a phenomenon also characteristic for fast proliferating cancer cells (*11, 14*). In addition, cancer cells are often endowed with high intrinsic ROS production and constitutive activation of the NF-κB pathway (*15-18*). Interestingly, an up-regulated glucose metabolism under hyperglycemic and hypoxic conditions was shown to induce mitochondrial ROS release in different cellular systems (*18-21*). These findings show that upon T cell activation the mitochondrial respiratory chain switches from an ATP producing to a signaling machinery.

During the experiments resulting in the present invention the metabolic changes accompanying the generation of the oxidative signal (*i.e.* occurring within 1 h upon TCR or PMA triggering (*1, 3*)) in partially glycolytic proliferating cells - *in vitro* expanded peripheral human T cells (*13, 22*) and Jurkat T cells (23) - was studied. It could be shown that TCR-triggered activation of ADP-dependent glucokinase (ADPGK), an alternative, typically archaeal glycolytic enzyme, mediates the generation of the oxidative signal. ADPGK-driven increase in glycolytic flux coincides with TCR-induced glucose uptake, down-regulation of mitochondrial respiration and deviation of glycolysis towards mitochondrial glycerol-3-phosphate (GPD) shuttle, *i.e.* a Warburg metabolic shift. The activation of respiratory chain-associated GPD2 results in hyper-reduction of ubiquinone and ROS release from mitochondrial complex I. In parallel, mitochondrial bioenergetics and ultrastructure are altered. Since the oxidative signal drives NF-κB-mediated gene expression, downregulation of ADPGK and GPD2 inhibits NF-κB induction, while ADPGK over-expression potentiates it. Interestingly, an enhanced expression of ADPGK and GPD2 in chronic lymphocytic leukemia (CLL) cells suggests a tumorigenic function. The described novel signaling and metabolic pathway may have profound meaning for tumorgenesis and may provide new means for therapy.

### Brief description of the drawings

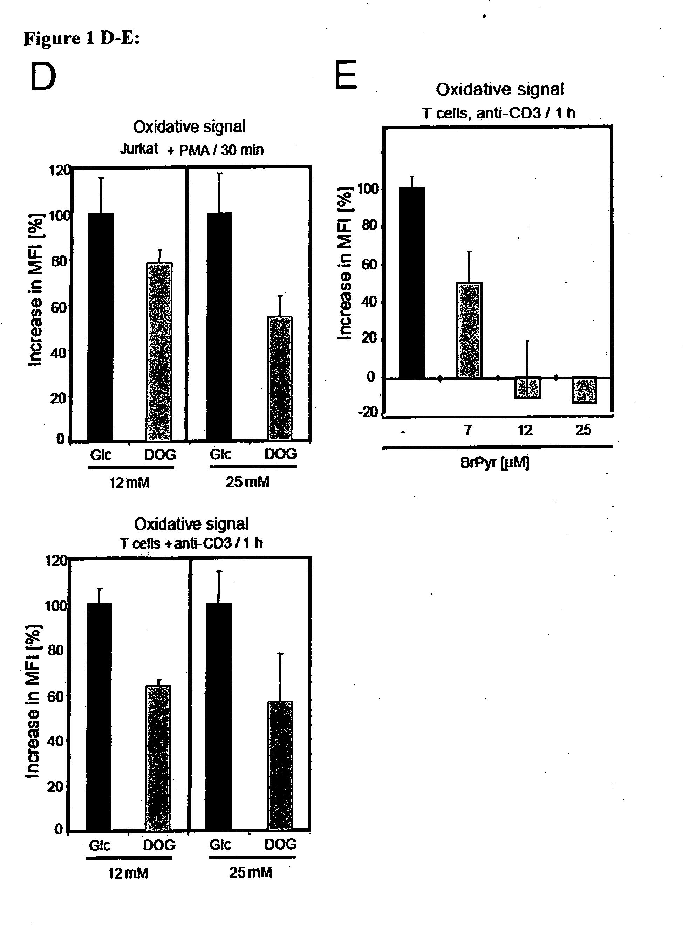

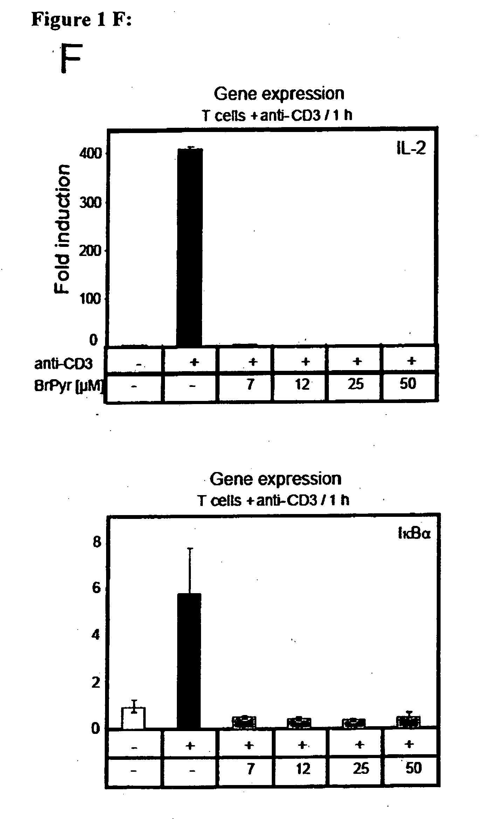

Figure 1
   **(A)** Respiratory rate of *in vitro* expanded peripheral **T** cells were monitored upon stimulation with soluble anti-CD3 antibody or PMA. Decrease in calculated "mitochondrial respiratory rate" between "control" state (15 min preceding induction) and "stimulated" state (indicated time intervals); (n=3) experiments (*i.e*. donors).
   **(B)** Uptake of D-[3-³H] glucose measured in **T** cells 1 h after activation (as in **A**). Results are shown as [%] of control (GAM - for anti-CD3; untreated - for PMA), (n=8) experiments +/-SD.
   (**C**) Intracellular ATP content of **T** cells activated (as in **A** and **B**), results of (n=3) experiments +/- SD.
   (**D-J**) Cells were 1 h stimulated with plate-bound CD3 antibody (30 pg/ml) or PMA (10 ng/ml). **D** and **E**, Stimulation-induced oxidative signal measured after pre-incubation: (**D**) in HANKS buffer + 110 µg/ml pyruvate + Glc/DOG (30 min) or (**E**) in culture medium + BrPyr (20 min). **F**, IL-2 and IκBα expression was determined in T cells treated as in (**E**) - representative triplicate measurement. **G**, Cells were snap-frozen, permeabilized and steady-state enzymatic activities of ETC complexes were measured. Results for (n=8, upper panel) or (n=4, lower panel) experiments are shown as [%] of untreated control (set to 100%, dashed line) +/- SD. **H** and **I**, Cells were pre-treated (20 min) with BIM. In (**H**) oxidative signal generation was measured and representative triplicated measurement +/- SD is shown. In (**I**) steady-state enzymatic activities were measured and presented as in (**G**). **J**, Electron flux from complex **I** (CI)/complex II (CII) to complex III (CIII) was measured in permeabilized cells or mitochondrial fractions. Data for (n) experiments are presented +/- SD (as in **G**). **K**, Cellular extracts were separated and analyzed by HPLC. Change in ubiquinone redox status presented as [%] difference between ubiquinonol/-one ratio of control cells (set to 0) and activated cells. Data are presented as mean +/-SD for (n) experiments (i.e. donors). Student's t test: p < 0.001 (***); p < 0.01 (**); p < 0.05 (*).

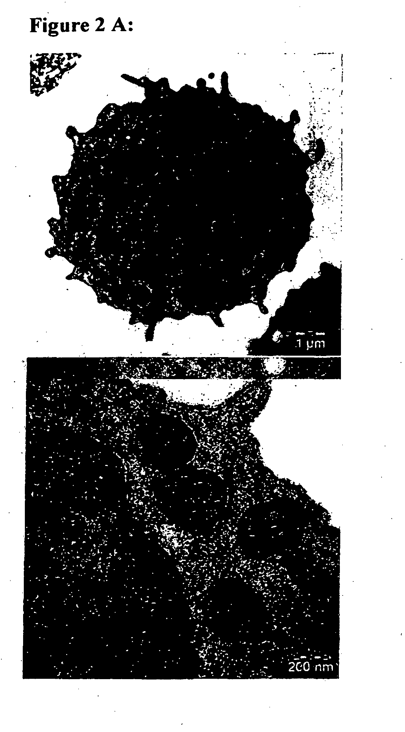

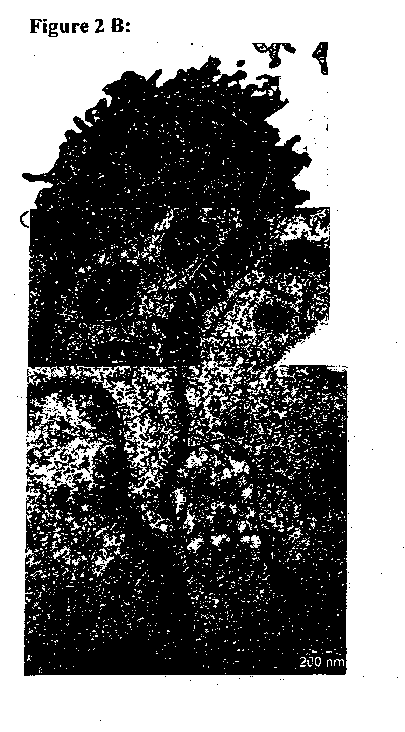

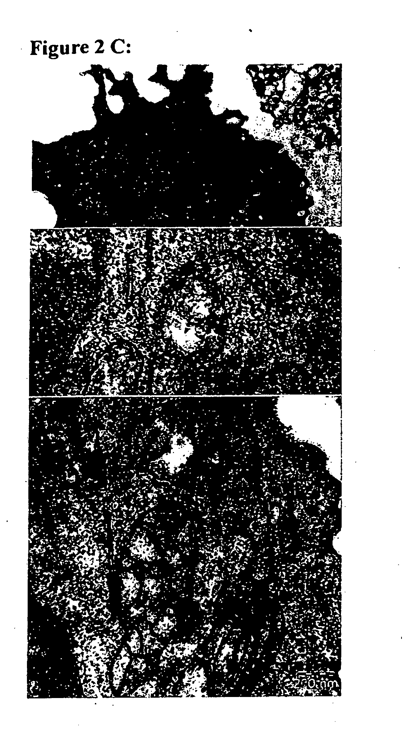

Figure 2: Rapid ultrastructural changes of mitochondria upon T cell activation
   **(A-C)** *In vitro* expanded peripheral human T cells were left untreated (**A**) or 1h activated (**B**, **C**) with PMA (10 ng/ml) (**B**) or plate-bound anti-CD3 antibody (30 µg/ml) (**C**). Cells were fixed, stained and subjected to electron microscopy. Representative images are presented. Scale bars: 1 µM (magnification 7.000x) or 200 nM (magnification 50.000X).

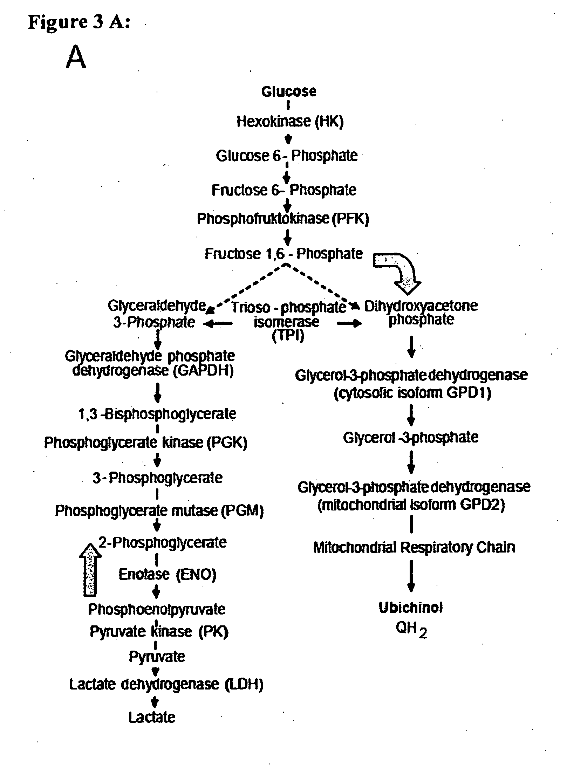

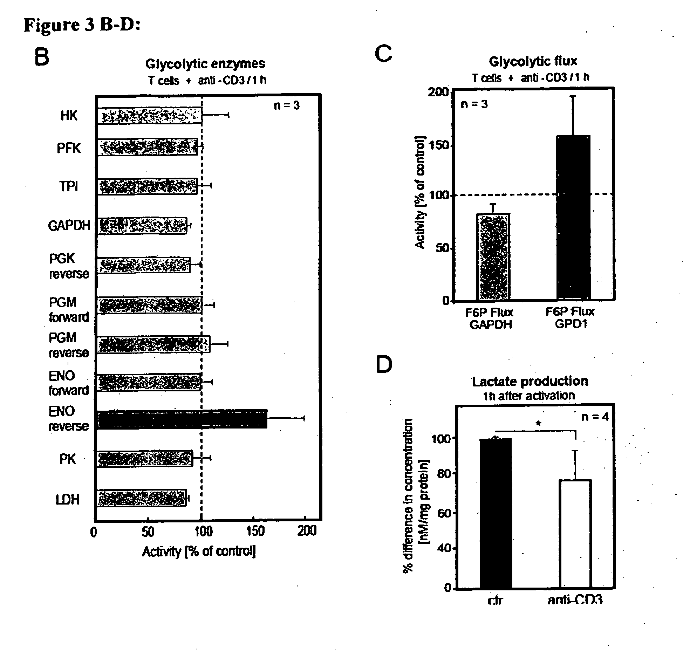

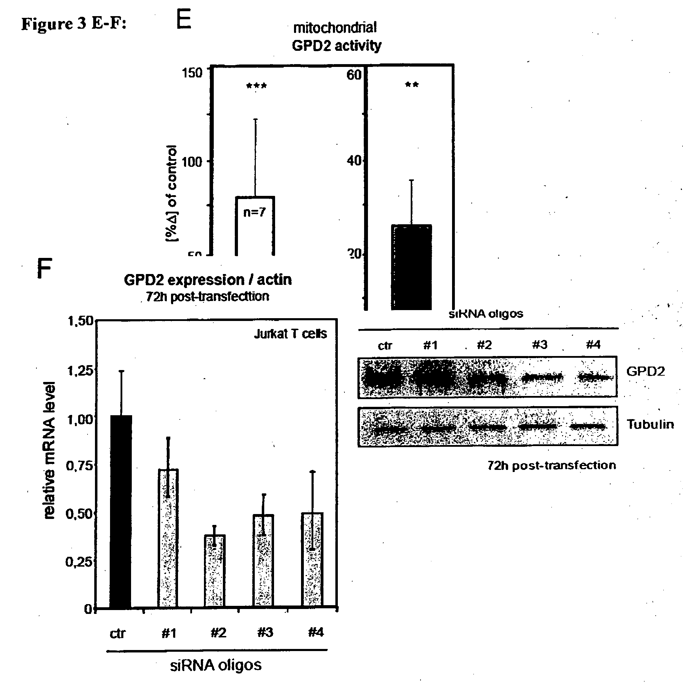

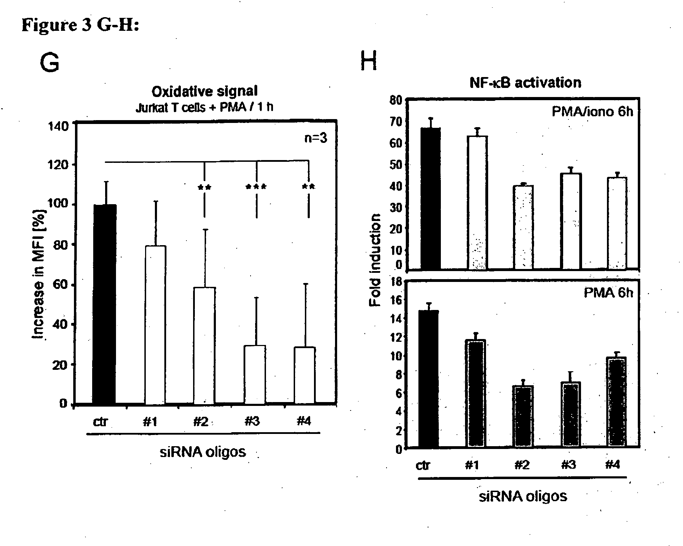

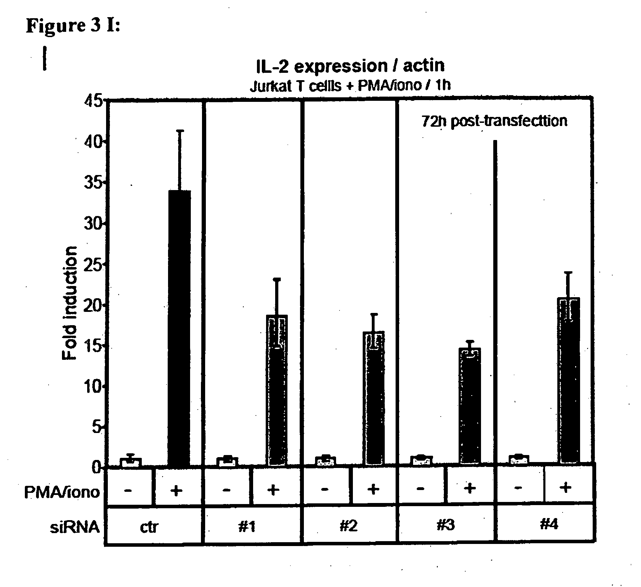

Figure 3: T cell activation diverts the glycolytic flux towards mitochondrial GPD shuttle
   (**A**) The glycolytic pathway (block arrows indicate the diverted metabolic flow). *In vitro* expanded T cells were 1h stimulated with plate-bound CD3 antibody (30 µg/ml) (**B**, **C**, **E**), GAM crosslinked anti-CD3 antibody (10 µg/ml) (**D,** control - GAM only) or PMA (10 ng/ml) (**E**, **G**).
   (**B**) Status of glycolytic enzymes upon T cell activation (acronyms - see **A**). Results are shown as [%] of untreated control (set to 100%, dashed line); (n=3) experiments (i.e. donors) +/- SD.
   (**C**) Fructose-6-phosphate (F6P) metabolic flux towards GPD1/GAPDH measured in the cytosol of activated T cells. Activation and data presentation as in (**B**).
   (**D**) Change of lactate concentration after activation of expanded T cells; (n) experiments +/- SD.
   (**E**) Change of GPD2 activity in mitochondrial fractions of activated T cells +/- pre-treatment with BIM., shown as [%] of non-stimulated control; (n) experiments (i.e. donors) +/- SD.
   (**F**) Real time qRT-PCR (left panel) and WB (right panel) analysis of siRNA-mediated GPD2 knock-down in Jurkat cells.
   (**G-I**) GPD2 expression was knocked-down in Jurkat cells (**G**, **I**) or stable NF-κB-luciferase reporter Jurkat cells (**H**). Cells were activated with PMA (10 ng/ml) +/- Iono (1 µM) and ROS (**G**), NF-κB activation (**H**) or induction of IL-2 expression (**I**) were assayed. Data are presented as representative experiments or mean of (n) experiments +/- SD. Student's t test: p < 0.001 (***); p < 0.01 (**); p < 0.05 (*).

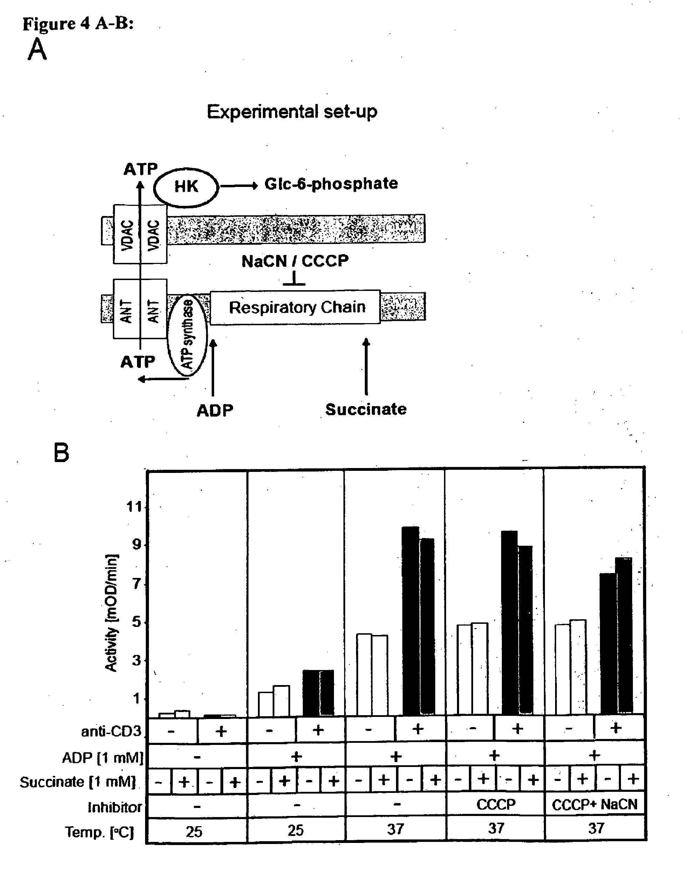

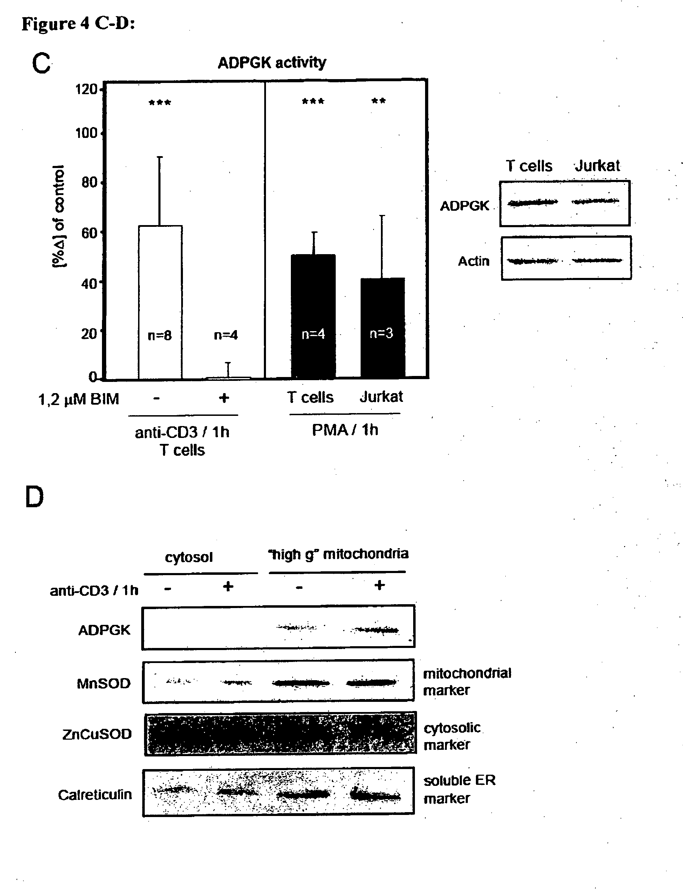

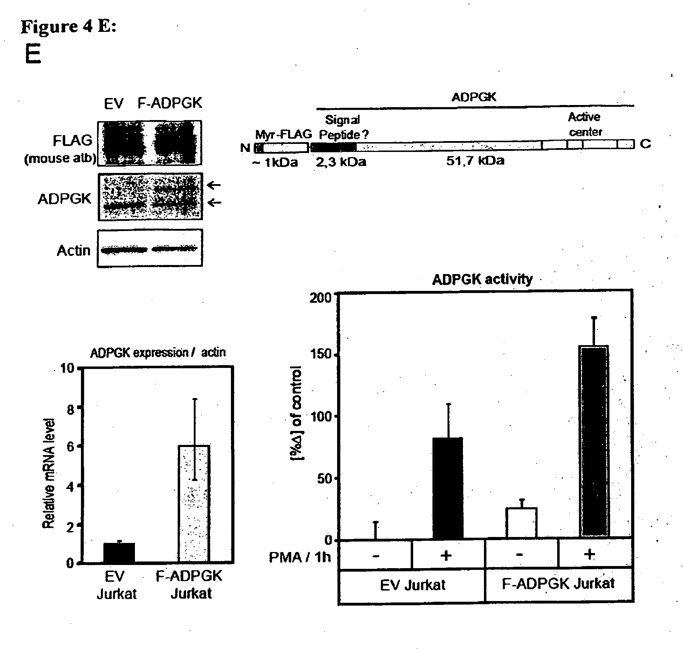

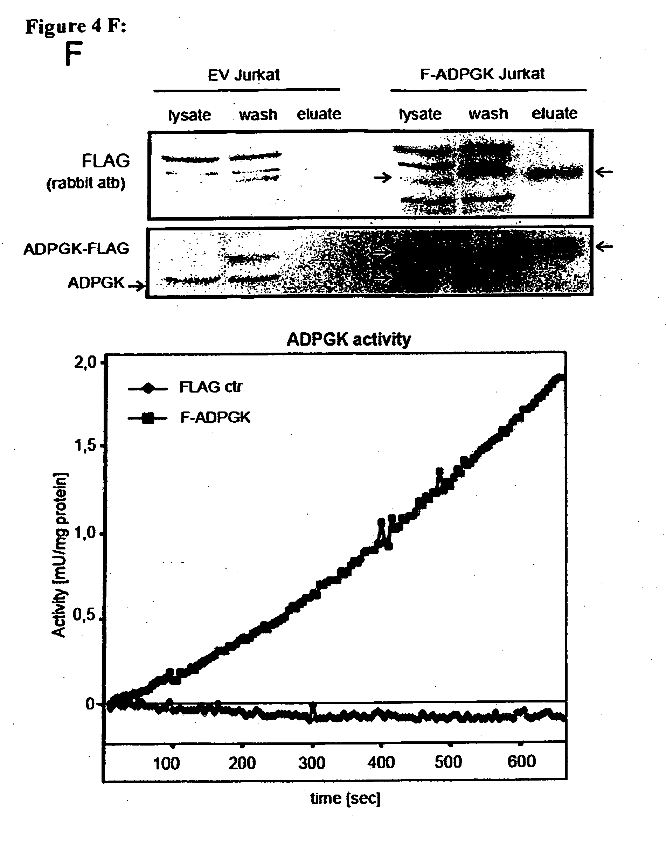

Figure 4: T cell activation triggers ADPGK enzymatic activity. Cells were 1h stimulated with a plate-bound CD3 antibody (30 µg/ml) (B-D) or PMA (10 ng/ml) (C, E)
   (**A**) An experimental set-up to measure respiration-coupled hexokinase (HK) activity in expanded T cells' mitochondria ("high g" fraction).
   (**B**) Results of experiment depicted in (**A**); CCCP (0,125 µM), NaCN (10 mM).
   (**C**) Left panel, ADPGK activity measured in the mitochondria ("high g" fraction) of activated T cells or Jurkat cells +/pre-treatment with BIM (20 min). Results for (n) experiments (*i.e*. donors) are shown as [%] of untreated control +/- SD. Right panel, WB of human ADPGK in total lysates of expanded human T cells or Jurkat cells.
   (**D**) WB analysis of mitochondrial ("high g") and cytosolic fractions of activated, expanded T cells.
   (**E**) Expression of the FLAG-ADPGK construct in Jurkat cells (F-ADPGK Jurkat, EV Jurkat - empty vector control cells). Left panel, WB (arrows - F-ADPGK and ADPGK proteins) and real-time qRT-PCR analysis of (F-)ADPGK content and expression. Upper right panel, a retroviral construct. Lower right panel, ADPGK activity in "ER-enriched" fractions of activated F-ADPGK and EV Jurkat cells. Representative triplicated experiment +/- SD is presented as in **C** (activity for samples of untreated EV Jurkat cells set to 0).
   (**F**) Upper panel, WB of immunoprecipitated F-ADPGK (IP; arrows: F-ADPGK and ADPGK proteins; FLAG WB - rabbit anti-FLAG antibodies; ADPGK WB - mouse antibodies, upper band on ADPGK WB in EV Jurkat "wash" line - H chain of mouse anti-FLAG (M2) antibody). Lower panel, ADPGK activity in immunoprecipitates ("eluate"). Student's t test: p < 0.001 (***); p < 0.01 (**).

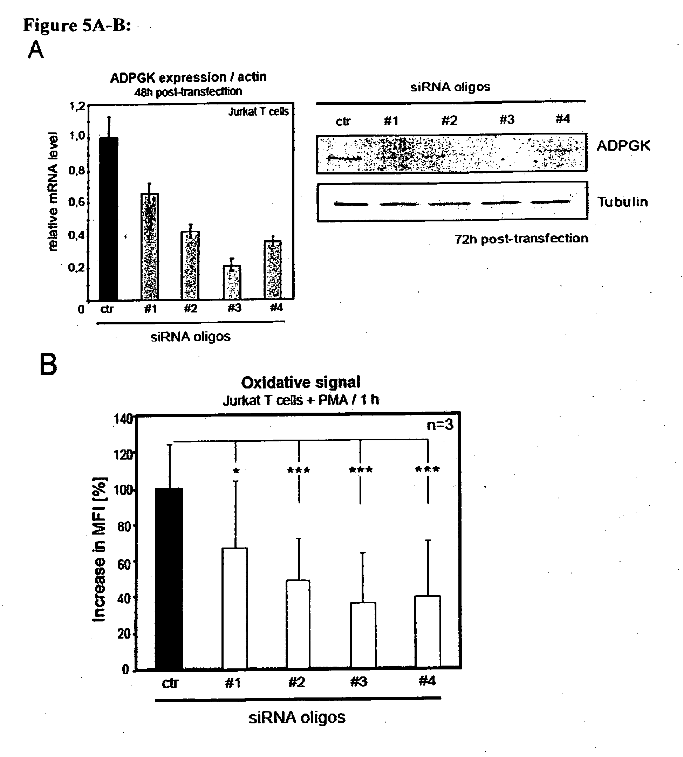

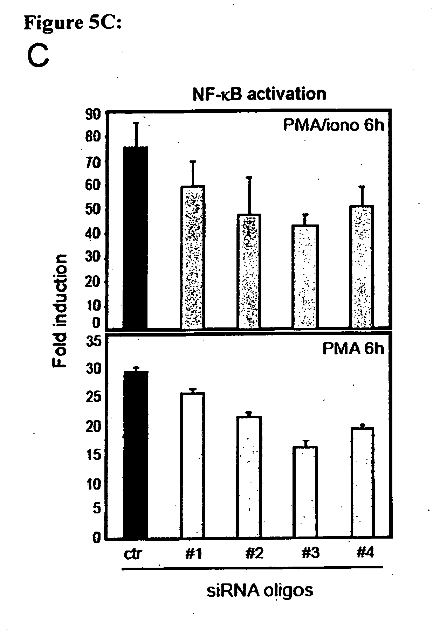

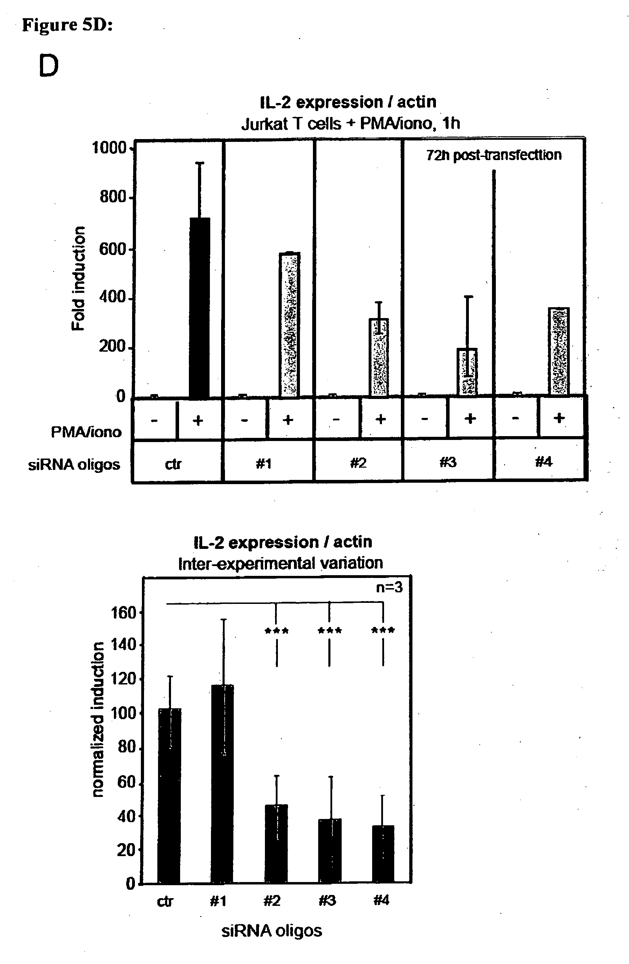

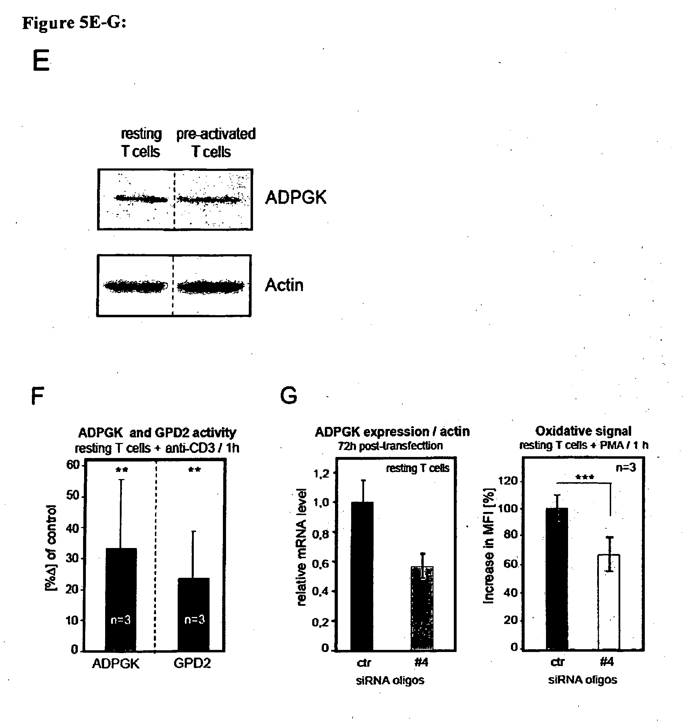

Figure 5: Lowered ADPGK content inhibits the activation-induced oxidative signal generation and NF-κB response
   (**A**) Jurkat cells were siRNA-transfected, and ADPGK expression and content were analyzed by real time qRT-PCR and WB.
   (**B-D**) After knock-down of ADPGK, Jurkat cells (**B, D**) or stable NF-κB-luciferase reporter Jurkat cells (C) were activated with PMA (10 ng/ml) +/- Iono (1 µM) and oxidative signal generation (**B**), NF-κB activation (**C**), or induction of IL-2 expression (**D**) were analyzed. Representative experiments performed in triplicate (**C** and **D**, upper panel) or inter-experimental comparison (**B** and **D**, lower panel) are presented.
   (**E**) ADPGK protein levels in "resting" and *in vitro* expanded ("pre-activated") human T cells.
   (**F**) The change of ADPGK and GPD2 activity in "high g" mitochondrial fractions of "resting" human T cells 1 h stimulated with plate-bound anti-CD3 antibodies (30 µg/ml). Results for (n) experiments (i.e. donors) are shown as [%] of non-stimulated controls +/- SD.
   (**G**) Left panel, a real time qRT-PCR analysis of siRNA-mediated knock-down of ADPGK expression in "resting" T cells. Right panel, after ADPGK knock-down T cells were stimulated with PMA (10 ng/ml) for 1 h and the oxidative signal generation was measured (n=3 experiments). Student's t test: p < 0.001 (***); p < 0.01 (**); p < 0.05 (*).
Figure 6
   (**A-C**) Jurkat cells stably expressing FLAG-ADPGK (F-ADPGK) protein (**A, C**) or Jurkat cells (**C**) and NF-κB-luciferase reporter Jurkat cells (**B**) transiently over-expressing WT-ADPGK protein for 24 h (**B, C**) were activated with PMA (10 ng/ml) +/-Iono (10 µM) for 1 h (**A, C**) or 6 h (**B**). Thereafter, (**A**) oxidative signal, (**B**) NF-κB activation, (**C**) induction of IL-2, IL-8 or IκBα expression were analyzed. Statistical comparison of (n) triplicated experiments is presented (response of EV control cells set to 100). **B**, WB analysis of transient WT-ADPGK overexpression.
   (**D**) Specific enzymatic activities of ADPGK and HK were measured in "high g" mitochondrial fractions of expanded human T cells at 25 °C, 30 °C, 35 °C and 42 °C. ADPGK activity at 25 °C set to 100%.
   (**E**) ADPGK and GPD2 gene expression in normal B cells (n=9) and CLL cells (n=10) was analyzed by real-time qRT-PCR. Values of "relative gene expression" levels (normalized to actin transcripts) from two experimental sets are presented as "z-scores". Student's t test: p < 0.001 (***); p < 0.01 (**), p < 0.05 (*), "outlier" values are marked with circles. (**F**)Scheme of the described pathway. Block arrows: changes in enzymatic activity, transport (glucose), ubiquinol content or direction of diverted metabolic flux.
Figure 7:
   (**A**) Mitochondrial oxygen consumption rate depicted as Δ CO₂ [ml O₂/1] of *in vitro* expanded human peripheral **T** cells prior and after activation. Arrows - stimulation with soluble anti-CD3 (10 µg/ml + 2 µg/ml GAM) antibodies or PMA (10 ng/ml). Representative recordings for three independent experiments are shown.
   **(B and C)** Jurkat T cells or primary human T cells were stained with H₂DCF-DA and treated with glucose (Glc), 2-deoxy-glucose (DOG) or 3-bromopyruvate (BrPyr) then activated as described in Figure 1D and E. **B**, Intracellular oxidative status was estimated by staining intensity (relative MFI units). **C**, anti-CD3 oxidative signal measured as in Figure 1E. **D**, Cells were treated and stimulated as in Figure 1F and IL-4 gene expression was assayed by real time qRT-PCR.
Figure 8
   **(A)** *In vitro* expanded human peripheral T cells were stimulated with plate-bound anti-CD3 antibody (30 µg/ml) for indicated time periods. Next, the intracellular content of selected respiratory chain components was analyzed by WB.
   (**B**) Left panel, mitochondrial fractions of anti-CD3 treated T cells (as in **A**; 2 donors) were solubilized in buffer containing 0,01% digitonin and resolved by BN-PAGE. Right lanes - influence of digitonin on migratory behavior of complex III (BC1 complex). Dashed frame - bends for complex III/GPD2. Right panel, molecular mass marker and WB analysis of BN-PAGE gel for GPD2.
Figure 9
   **(A)** *In vitro* expanded human peripheral T cells were stained with H₂DCF-DA, pre-treated for 15 min with respective inhibitor and stimulated by plate-bound anti-CD3 antibody (30 µg/ml) or PMA (10 ng/ml) for 1 h. Next, oxidative signal generation was measured by FACS.
   (**B**) Specific activity of mitochondrial respiratory chain complexes in mitochondria isolated from *in vitro* expanded human peripheral T cells or mouse heart muscle tissue.
Figure 10
   **(A** and **B**) *In vitro* expanded human T cells were pre-treated with bis-indolyl-maleimidate I (BIM) for 20 min and stimulated with plate-bound anti-CD3 antibodies (30 µg/ml) for 1 h. Mitochondrial "high g" fractions were assessed for HK1 and HK2 content (**A**) or HK activity (**B**). In **A**, arrows indicate WB bands for anti-HK2 antibodies. In **B**, results for (n) independent experiments (*i.e*. donors) are shown as [%] of untreated control (set to 100%, dashed line) +/- inter-experimental SD, *t* test p > 0,05 (not significant).
Figure 11
   (**A**) Jurkat T cells stably expressing FLAG-ADPGK protein (F-ADPGK) and (**B**) *in vitro* expanded peripheral human T cells (stimulated +/- plate-bound anti-CD3 antibody, 30 µg/ml) were subjected to sub-cellular fractionation into cytosolic, "mitochondria-enriched" and "ER-enriched" fractions. ADPGK contents was analyzed by WB; MnSOD - mitochondrial marker, calreticulin - soluble ER marker, ZnCuSOD - cytoplasmic marker. **B**, upper band on ADPGK WB - H chain of mouse anti-CD3 antibody used for stimulation.
Figure 12:
   Jurkat T cells after siRNA-mediated down-regulation of ADPGK expression (72 h post-transfection) were stimulated for 1 h with PMA (10ng/ml) and ionomycin (Iono, 1 µM). Induction of IL-8 and IκBα gene expression (normalized to actin transcripts) was analyzed by real-time qRT-PCR.
Figure 13:
   Basal IL-2, IκBα and IL-8 gene expression levels in unstimulated Jurkat T cells stably expressing FLAG-ADPGK protein (F-ADPGK) or transiently over-expressing WT ADPGK protein (WT-ADPGK, 24 h post-transfection). Transcript levels were normalized to actin expression and compared to expression levels of respective empty vector (EV) control cells (set to 1, dashed line). Results for (n) independent experiments +/inter-experimental SD are shown.

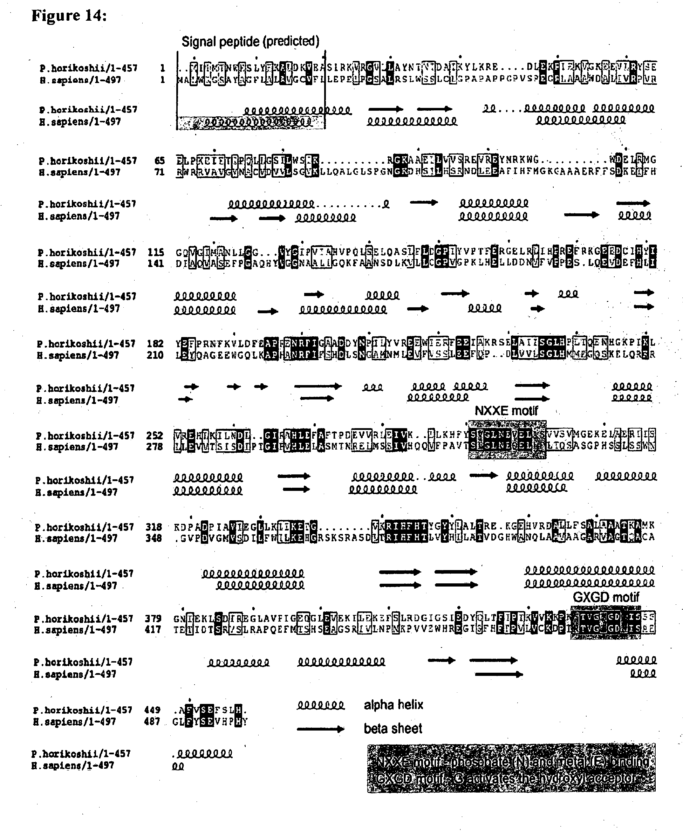

Figure 14: A comparison of amino acid (aa) sequences and secondary structures of *Homo sapiens* and *Pyroccocus horikoshii* OT3 ADPGK proteins
   For *H. sapiens -* secondary structure prediction by Jpred v.3 (*14*) (UniProtKB canonical sequence Q9BRR6-1), for *P*. *horikoshii -* secondary structure motives based on crystal structure (Protein Data Bank code: 1L2L) (*15*) common for known crystal structures of thermophilic ADPGK proteins (*16*). Grey squares - homologous aas; blank squares - similar aas; NXXD and GXGD motives - conserved active center; frame - a predicted signal peptide.

Thus, the present invention relates to a compound capable of reducing or inhibiting (a) the biological activity of ADP-dependent glucokinase (ADPGK) or (b) the expression of the gene encoding ADPGK for use in a method of treating a neoplasm, an autoimmune disease or Graft-versus-Host-Disease (GvHD).

The reduction or inhibition of the biological activity can be effected by direct interaction or binding of a compound to ADPGK or by indirect interaction, e.g., by interacting with a compound that is associated with the biological activity of ADPGK. The reduction or inhibition of the biological activity can also be achieved by the application of altered, e.g., inactive forms of ADPGK preferably in excess.

Examples of suitable compounds reducing or inhibiting the biological activity of ADPGK, e.g., immune suppressive modulators, or the expression of the corresponding gene with the aim to get a therapeutic effect are:
- an antisense oligonucleotide,
- siRNA reducing or inhibiting the expression of the gene encoding ADPGK,
- an antibody directed against ADPGK or a fragment thereof having the same specificity,
- an inactive version of ADPGK, or
- a polynucleic acid encoding an inactive version of ADPGK.

Further compounds suitable for the purposes of the present invention and methods how to identify/select such compounds are in more detail described below.

Preferably, in a pharmaceutical composition, such compounds as described above and below are combined with a pharmaceutically acceptable carrier. "Pharmaceutically acceptable" is meant to encompass any carrier, which does not interfere with the effectiveness of the biological activity of the active ingredient and that is not toxic to the patient to which it is administered. Examples of suitable pharmaceutical carriers are well known in the art and include phosphate buffered saline solutions, water, emulsions, such as oil/water emulsions, various types of wetting agents, sterile solutions etc.. Such carriers can be formulated by conventional methods and the active compound can be administered to the subject at an effective dose.

An "effective dose" refers to an amount of the active ingredient that is sufficient to affect the course and the severity of the disease, e.g., neoplasia, leading to the reduction or remission of such a pathology. An "effective dose" useful for treatment may be determined using methods known to one skilled in the art.

Administration of the suitable compositions may be effected by different ways, e.g. by intravenous, intraperitoneal, subcutaneous, intramuscular, topical or intradermal administration. The route of administration, of course, depends on the kind of therapy and the kind of compound contained in the pharmaceutical composition. The dosage regimen will be determined by the attending physician and other clinical factors. As is well known in the medical arts, dosages for any one patient depends on many factors, including the patient's size, body surface area, age, sex, the particular compound to be administered, time and route of administration, the kind of therapy, general health and other drugs being administered concurrently.

The person skilled in the art can easily identify or generate compounds useful for the treatments of the present invention based on the knowledge of the amino acid sequences of ADPGK, and the nucleotide sequences of the genes encoding these proteins (ADPGK, nucleotide sequence: NCBI NM_031284.4 (496 aa isoform) + M_031284.2 (497 aa isoform); amino acid sequence: UniProtKB canonical sequence Q9BRR6-2 (496 aa isoform) + Q9BRR6-1 (497 aa isoform)).

In a further preferred embodiment of the present invention, the compound useful for reducing or inhibiting the expression of the gene encoding ADPGK is an antisense oligonucleotide or siRNA specific for said gene.

The generation of suitable antisense oligonucleotides includes determination of a site or sites within the ADPGK encoding gene for the antisense interaction to occur such that the desired effect, e.g., inhibition of the expression of the protein, will result. A preferred intragenic site is (a) the region encompassing the translation initiation or termination codon of the open reading frame (ORF) of the gene or (b) a region of the mRNA which is a "loop" or "bulge", i.e., not part of a secondary structure. If one or more target sites have been identified, oligonucleotides are chosen which are sufficiently complementary to the target, i.e., hybridize sufficiently well and with sufficient specificity, to give the desired effect. In the context of this invention, "hybridization" means hydrogen bonding, which may be Watson-Crick, Hoogsteen or reversed Hoogsteen hydrogen bonding, between complementary nucleoside or nucleotide bases. "Complementary" as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can make hydrogen bonds with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense compound does not need to be 100% complementary to that of its target nucleic acid to be specifically hybridizable. An antisense compound is specifically hybridizable when binding of the compound to the target DNA or RNA molecule interferes with the normal function of the target DNA or RNA to cause a loss of utility, and there is a sufficient degree of complementarity to avoid non-specific binding of the antisense compound to non-target sequences under conditions in which specific binding is desired, i.e., in the case of therapeutic treatment.

The skilled person can generate antisense compounds and siRNAs according to the present invention on the basis of the known DNA sequences for ADPGK.

"Oligonucleotide" refers to an oligomer or polymer of ribonucleic acid (RNA) or deoxyribonucleic acid (DNA) or mimetics thereof. This term includes oligonucleotides composed of naturally-occurring nucleobases, sugars and covalent internucleoside (backbone) linkages as well as oligonucleotides having non-naturally-occurring portions which function similarly. Such modified or substituted oligonucleotides are often preferred over native forms because of desirable properties such as, for example, enhanced cellular uptake, enhanced affinity for nucleic acid target and increased stability in the presence of nucleases. While antisense oligonucleotides are a preferred form of the antisense compound, the present invention comprehends other oligomeric antisense compounds, including but not limited to oligonucleotide mimetics such as are described below. The antisense compounds in accordance with this invention comprise from about 8 to about 50 nucleobases (i.e. from about 8 to about 50 linked nucleosides). Particularly preferred antisense compounds are antisense oligonucleotides, even more preferably those comprising from about 15 to about 25 nucleobases. Antisense compounds include ribozymes, external guide sequences (EGS), oligonucleotides (oligozymes), and other short catalytic RNAs or catalytic oligonucleotides which hybridize to the target nucleic acid and inhibit its expression.

Alternatively, the compound of the invention is a vector allowing to transcribe an antisense oligonucleotide of the invention, e.g., in a mammalian host. Preferably, such a vector is a vector useful for gene therapy. Preferred vectors useful for gene therapy are viral vectors, e.g. adenovirus, herpes virus, vaccinia, or, more preferably, an RNA virus such as a retrovirus. Even more preferably, the retroviral vector is a derivative of a murine or avian retrovirus. Examples of such retroviral vectors which can be used in the present invention are: Moloney murine leukemia virus (MoMuLV), Harvey murine sarcoma virus (HaMuSV), murine mammary tumor virus (MuMTV) and Rous sarcoma virus (RSV). Most preferably, a non-human primate retroviral vector is employed, such as the gibbon ape leukemia virus (GaLV), providing a broader host range compared to murine vectors. Since recombinant retroviruses are defective, assistance is required in order to produce infectious particles. Such assistance can be provided, e.g., by using helper cell lines that contain plasmids encoding all of the structural genes of the retrovirus under the control of regulatory sequences within the LTR. Suitable helper cell lines are well known to those skilled in the art. Said vectors can additionally contain a gene encoding a selectable marker so that the transduced cells can be identified. Moreover, the retroviral vectors can be modified in such a way that they become target specific. This can be achieved, e.g., by inserting a polynucleotide encoding a sugar, a glycolipid, or a protein, preferably an antibody. Those skilled in the art know additional methods for generating target specific vectors. Further suitable vectors and methods for in vitro- or in vivo-gene therapy are described in the literature and are known to the persons skilled in the art; see, e.g., WO 94/29469 or WO 97/00957.

In order to achieve expression only in the target organ the DNA sequences for transcription of the antisense oligonucleotides can be linked to a tissue specific promoter and used for gene therapy. Such promoters are well known to those skilled in the art.

Within an oligonucleotide structure, the phosphate groups are commonly referred to as forming the internucleoside backbone of the oligonucleotide. The normal linkage or backbone of RNA and DNA is a 3' to 5' phosphodiester linkage. Specific examples of preferred antisense compounds useful in the present invention include oligonucleotides containing modified backbones or non-natural internucleoside linkages. Oligonucleotides having modified backbones include those that retain a phosphorus atom in the backbone and those that do not have a phosphorus atom in the backbone. Modified oligonucleotide backbones which can result in increased stability are known to the person skilled in the art, preferably such modification is a phosphorothioate linkage.

A preferred oligonucleotide mimetic is an oligonucleotide mimetic that has been shown to have excellent hybridization properties, and is referred to as a peptide nucleic acid (PNA). In PNA compounds, the sugar-backbone of an oligonucleotide is replaced with an amide containing backbone, in particular an aminoethylglycine backbone. The nucleobases are retained and are bound directly or indirectly to aza nitrogen atoms of the amide portion of the backbone.

Modified oligonucleotides may also contain one or more substituted or modified sugar moieties. Preferred oligonucleotides comprise one of the following at the 2' position: OH; F; 0-, S-, or N-alkyl; 0-, S-, or N-alkenyl; 0-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. A particularly preferred modified sugar moiety is a 2'-O-methoxyethyl sugar moiety.

Oligonucleotides of the invention may also include nucleobase modifications or substitutions. Modified nucleobases include other synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine etc., with 5-methylcytosine substitutions being preferred since these modifications have been shown to increase nucleic acid duplex stability.

Another modification of the oligonucleotides of the invention involves chemically linking to the oligonucleotide one or more moieties or conjugates which enhance the activity, cellular distribution or cellular uptake of the oligonucleotide. Such moieties include lipid moieties such as a cholesterol moiety, cholic acid, a thioether, a thiocholesterol, an aliphatic chain, e.g., dodecandiol or undecyl residues, a phospholipid, a polyamine or a polyethylene glycol chain, or adamantane acetic acid, a palmityl moiety, or an octadecylamine or hexylamino-carbonyl-oxycholesterol moiety.

The present invention also includes antisense compounds which are chimeric compounds. "Chimeric" antisense compounds or "chimeras," in the context of this invention, are antisense compounds, particularly oligonucleotides, which contain two or more chemically distinct regions, each made up of at least one monomer unit, i.e., a nucleotide in the case of an oligonucleotide compound. These oligonucleotides typically contain at least one region wherein the oligonucleotide is modified so as to confer upon the oligonucleotide increased resistance to nuclease degradation, increased cellular uptake, and/or increased binding affinity for the target nucleic acid. An additional region of the oligonucleotide may serve as a substrate for enzymes capable of cleaving RNA:DNA or RNA:RNA hybrids. By way of example, RNase H is a cellular endonuclease which cleaves the RNA strand of an RNA:DNA duplex. Activation of RNase H, therefore, results in cleavage of the RNA target, thereby greatly enhancing the efficiency of oligonucleotide inhibition of gene expression. Consequently, comparable results can often be obtained with shorter oligonucleotides when chimeric oligonucleotides are used, compared to phosphorothioate deoxyoligonucleotides hybridizing to the same target region. Chimeric antisense compounds of the invention may be formed as composite structures of two or more oligonucleotides, modified oligonucleotides, oligonucleosides and/or oligonucleotide mimetics as described above. Such compounds have also been referred to in the art as hybrids or gapmers.

According to the present invention the compound of the present invention is used for the treatment of (i) a neoplasm or (b) an autoimmune disease, or Graft-versus-Host Disease (GvHD).

"Neoplasm" is an abnormal mass of tissue as a result of neoplasia. "Neoplasia" is the abnormal proliferation of cells. The growth of the cells exceeds and is uncoordinated with respect to the normal tissues around it. The growth persists in the same excessive manner, even after cessation of the stimuli. It usually causes a lump or tumor. Neoplasms may be benign, pre-malignant (carcinoma-in-situ) or malignant (cancer). The neoplasms to be treated according to the present invention comprise those which (over)express ADPGK . Thus, the determination of ADPGK in a neoplasm is an indication to start with a ADPGK inhibiting therapy. A neoplasm to be treated is particularly B cell chronic lymphocytic leukemia or any neoplasm characterized in that T cell activation shows a beneficial effect, i.e., tumors of the immune system and tumors characterized by constitutive activation of NF-kappaB and/or increased ROS levels. Examples of such tumors are Multiple Myeloma (MM), Diffuse large B cell Lymphoma, Acute Myelogenous leukemia (AML), Chronic myelogenous leukemia (CML), Adult T cell lymphoma (ATL), childhood/T cell acute lymphoblastic

leukemia (ALL/T-ALL), Hodgin Lymphoma (HL), Non-Hodgin Lymphoma, Malt lymphoma, Cutaneous T-cell lymphoma (CTCL), e.g., Sézary Syndrom (SS) and Hepatocellular Carcinoma (HCC).

Preferred autoimmune diseases that can be treated with a compound of the present invention are, e.g., rheumatism, Lupus erythematodes, Psoriasis, atopic dermatitis, multiple sclerosis, or diabetes.

Further examples of compounds capable of reducing or inhibiting the biological activity of ADPGK are (neutralizing) antibodies directed against these proteins or fragments thereof having substantially the same binding specificity or pseudo-substrates. The term "antibody", preferably, relates to antibodies which consist essentially of pooled monoclonal antibodies with different epitopic specificities, as well as distinct monoclonal antibody preparations. Monoclonal antibodies are made from an antigen containing, e.g., a fragment of ADPGK by methods well known to those skilled in the art (see, e.g., Köhler et al., Nature 256 (1975), 495). As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody, clear more rapidly from the circulation, and may have less non-specific tissue binding than an intact antibody. (Wahl et al., J. Nucl. Med. 24: 316-325 (1983)). Thus, these fragments are preferred, as well as the products of a FAB or other immunoglobulin expression library. Moreover, antibodies useful for the purposes of the present invention include chimerical, single chain, and humanized antibodies.

Alternatively, preferred compounds for the purposes of the invention are inactive versions of ADPGK or nucleic acid sequences encoding inactive versions of these proteins that can be introduced according to the approaches/vectors described above. Such inactive versions can be generated according to well known methods of mutagenesis. Such compounds can have a therapeutic effect in the human body by displacing their functionally active counterpart, in particular when applied in excess. Analyses of potentially inactive versions of ADPGK can be carried out by assaying the (reversible) transamination of branched-chain L-amino acids to branched-chain alpha-keto acids, e.g., by determining the production of glutamate. Suitable assays are described in the literature.

The present invention also relates to a method for identifying a compound capable of modulating the biological activity of ADPGK and/or the expression of ADPGK, comprising the steps of:
(a) incubating a candidate compound with a test system comprising ADPGK or the gene encoding ADPGK; and
(b) assaying a biological activity of ADPGK;
wherein an increase or reduction of the biological activity of ADPGK, preferably compared to a test system in the absence of said test compound, is indicative of the presence of a candidate compound having the desired property.

The increase or reduction of the biological activity of ADPGK can be assayed by determining the concentration of the protein, e.g., by use of a specific antibody or by directly determining the enzymatic activity of the protein, e.g., by determining the change of the concentration of a specific substrate or end product as described, e.g., in Example 1.

Examples of such candidate molecules include antibodies, oligonucleotides, proteins, or small molecules. Such molecules can be rationally designed using known techniques.

Preferably, said test system used for screening comprises substances of similar chemical and/or physical properties, most preferably said substances are almost identical. The compounds which can be prepared and identified according to a use of the present invention may be expression libraries, e.g., cDNA expression libraries, peptides, proteins, nucleic acids, antibodies, small organic compounds, ligands, hormones, peptidomimetics, PNAs or the like.

WO 98/25146 describes further methods for screening libraries of complexes for compounds having a desired property, especially, the capacity to agonize, bind to, or antagonize a polypeptide or its cellular receptor. The complexes in such libraries comprise a compound under test, a tag recording at least one step in synthesis of the compound, and a tether susceptible to modification by a reporter molecule. Modification of the tether is used to signify that a complex contains a compound having a desired property. The tag can be decoded to reveal at least one step in the synthesis of such a compound. Other methods for identifying compounds which interact with ADPGK or nucleic acid molecules encoding such molecules are, for example, the in vitro screening with the phage display system as well as filter binding assays or "real time" measuring of interaction.

It is also well known to the person skilled in the art, that it is possible to design, synthesize and evaluate mimetics of small organic compounds that, for example, can act as a substrate or ligand to ADPGK.

All these methods can be used in accordance with the present invention to identify a compound modulating, e.g., reducing or inhibiting the biological activity of ADPGK or their expression.

The gene encoding ADPGK can also serve as a target for the screening of activators or inhibitors, e.g., immune suppressive modulators. Inhibitors may comprise, for example, proteins that bind to the mRNA of the genes encoding ADPGK, thereby destabilizing the native conformation of the mRNA and hampering transcription and/or translation. Furthermore, methods are described in the literature for identifying nucleic acid molecules such as a RNA fragment that mimics the structure of a defined or undefined target RNA molecule to which a compound binds inside of a cell resulting in the retardation of the cell growth or cell death; see, e.g., WO 98/18947 and references cited therein. These nucleic acid molecules can be used for identifying unknown compounds of pharmaceutical interest, and for identifying unknown RNA targets for use in treating a disease. These methods and compositions can be used for identifying compounds useful to reduce expression levels of ADPGK.

The compounds which can be tested and identified according to the method of the invention may be expression libraries, e.g., cDNA expression libraries, peptides, proteins, nucleic acids, antibodies, small organic compounds, hormones, peptidomimetics, PNAs or the like. Furthermore, genes encoding a putative regulator of ADPGK and/or which exert their effects up- or downstream of ADPGK may be identified using insertion mutagenesis using, for example, gene targeting vectors known in the art. Said compounds can also be functional derivatives or analogues of known inhibitors, substrates or modulators. Such useful compounds can be for example transacting factors which bind to ADPGK or regulatory sequences of the gene encoding it. Identification of transacting factors can be carried out using standard methods in the art. To determine whether a protein binds to the protein itself or regulatory sequences, standard native gel-shift analyses can be carried out. In order to identify a transacting factor which binds to the protein or regulatory sequence, the protein or regulatory sequence can be used as an affinity reagent in standard protein purification methods, or as a probe for screening an expression library. The identification of nucleic acid molecules which encode polypeptides which interact with ADPGK can also be achieved, for example, by use of the so-called yeast "two-hybrid system". In this system ADPGK is linked to the DNA-binding domain of the GAL4 transcription factor. A yeast strain expressing this fusion polypeptide and comprising a lacZ reporter gene driven by an appropriate promoter, which is recognized by the GAL4 transcription factor, is transformed with a library of cDNAs which will express plant proteins or peptides thereof fused to an activation domain. Thus, if a peptide encoded by one of the cDNAs is able to interact with the fusion peptide comprising a peptide of ADPGK, the complex is able to direct expression of the reporter gene. In this way, ADPGK and the gene encoding ADPGK can be used to identify peptides and proteins interacting with ADPGK. It is apparent to the person skilled in the art that this and similar systems may then further be exploited for the identification of inhibitors.

Finally, the present application describes a method of selecting a therapy modality for a patient afflicted with a disease as characterized above, comprising
(a) obtaining a sample from said patient; and
(b) determining the level and/or activity of ADPGK;
whereby the mode of treatment depends on the level and/or activity of ADPGK.

Preferably, the level of ADPGK is determined on the protein level using an antibody that specifically binds to ADPGK or by determining a biological activity of the protein.

In the method which relates to the selection of a therapy modality for a patient, the terms "therapy modality" or "mode of treatment" refer to a timely sequential or simultaneous administration of compounds having an effect on the level/activity of ADPGK, respectively, provided that the results of the method of the invention indicate that the disease is associated with an aberrant level/activity of ADPGK.

The below examples explain the invention in more detail.

### Example 1

### Materials and Methods

### (A) Chemicals

If not stated differently all reagents and enzymes used were supplied by Sigma-Aldrich (Munich, Germany). Dichlorodihydrofluorescein diacetate (H₂DCF-DA) was obtained from Invitrogen (Carlsbad, CA, USA). Iono was purchased from Merck (Darmstadt, Germany). Primary antibodies for Western blot (WB) were: from Sigma-Aldrich (Munich, Germany) - mouse monoclonal anti-FLAG (M2), rabbit polyclonal anti-FLAG, mouse monoclonal anti-γ-tubulin, rabbit polyclonal anti-GPD2 (Human Protein Atlas Antibodies); from Cell Signaling (Denvers, MA, USA) - rabbit polyclonal anti-HK1 and rabbit polyclonal anti-HK2; from Abcam (Cambridge, UK) - mouse monoclonal anti-human ADPGK (1E4); from Santa Cruz Biotechnology (Santa Cruz, CA, USA) - goat polyclonal anti-SOD1; from Milipore (Darmstadt, Germany) - rabbit polyclonal anti-SOD2, from ABR (Golden, CO, USA)- rabbit polyclonal anti-calreticulin; from GeneTex (Irvine, CA, USA) - rabbit polyclonal anti-β-actin and from Thermo Scientific (Bonn, Germany) - mouse monoclonal anti-PHB1 antibody (II-14-10). Content of mitochondrial respiratory complexes was analyzed using Total OXPHOS WB Antibody Cocktail from Mito Sciences (Eugene, OR, USA). FITC-conjugated anti-CD3 and anti-CD20 antibodies were from Becton Dickinson (Heidelberg, Germany). Cross-linking polyclonal goat antimouse antibody (GAM) was obtained from Southern Biotech (Birmingham, AL, USA). Monoclonal mouse antibody (OKT3) against human CD3 was prepared as described (*1*).

### (B) Isolation of human peripheral T cells and B cells

Generally, human peripheral blood T lymphocytes were purified as described previously (1). Human peripheral blood B lymphocytes were prepared from the Ficoll gradient inter-phase ring by negative MACS sorting ("B Cell Isolation Kit II", Miltenyi, Bergisch Gladbach, Germany). Homogeneity of the prepared T and B cells was verified by staining with FITC-conjugated anti-CD3 or anti-CD20 antibodies followed by FACS analysis and was estimated to be > 93% (B cells) and > 90% (T cells).

### (C) Patients

Informed consent was obtained from all subjects before inclusion. 9 out of 10 patients did not receive treatment prior to the therapy. Patient data (age, gender and genetic analysis of CLL samples) can be found in Table 1. B cells from CLL patients were isolated by Ficoll-Paque centrifugation (GE Healthcare; Chalfont St. Giles, UK). Purity was estimated to be > 90%. The study was conducted according to the ethical guidelines of the German Cancer Research Center (DKFZ, Heidelberg) and the Helsinki Declaration, and approved by the ethics committee II of the Ruprecht-Karls-University of Heidelberg, Germany.

### (D) Cell culture

T cell line Jurkat J16-145 cells, J16 sub-clone (2), were cultured in RPMI 1640 (+ L-glutamine), 10% foetal calf serum (FCS). *Gaussia* luciferase NF-κB reporter J16-145 cells were cultured in IMDM, 10% FCS. Freshly isolated ("resting") or phytohemagglutinin (PHA)-activated and 6-8 days *in vitro* expanded ("pre-activated") peripheral human T cells were cultured at initial concentration of 2x10⁶ cells/ml in RPMI 1640 (+ L-glutamine), 10% FCS. For pre-activation-induced expansion, "resting" T cells were treated with 1 µg/ml PHA for 16 h, washed and subsequently cultured in the presence of 25 U/ml IL-2 for 6-8 days. These cells are referred to as "T cells" in the text, if not stated differently.

### (E) Determination of ROS generation

Cells were stained with H₂DCF-DA (5 µM) for 30 min. Next, cells were divided and 1 h stimulated with either plate-bound anti-CD3 antibody (30 µg/ml) or PMA (10 ng/ml). Treatment was terminated by ice-cold PBS and ROS generation was determined by FACS analysis (Canto II, Becton Dickinson). ROS generation was quantified as the increase in mean fluorescence intensity (MFI), calculated according to the following formula: increase in MFI (%) = [(MFI_{stimulated} -MFI_{unstimulated})/MFI_{unstimulated}] x 100 (3).

### (F) Digitonin-permeabilized cells

*In vitro* expanded T cells (at least 0,5 x 10⁸) were snap-frozen in liquid nitrogen and subsequently incubated on ice in ETC buffer (20 mM Tris-HCl pH 7.4, 250 mM sucrose, 50 mM KCl, 5 mM MgCl₂) with 0.015% digitonin for 30 min according to Chretien *et al.* (4) with minor modifications. Afterwards, cells were washed twice with ice-cold ETC buffer and subjected to assays.

### (G) Preparation of sub-cellular fractions

Cells were disrupted using a 27x1/2" needle in ice-cold ETC buffer and the homogenates were centrifuged at 600xg, 4°C for 10 min. For preparation of the "high g" mitochondrial (pellet) as well cytosolic (supernatant) fraction the 600xg supernatant was centrifuged at 11.000xg, 4°C for 20 min. For preparation of the "mitochondria-enriched" fractions, the 600xg supernatant was centrifuged 10 min, 4°C at 3,500xg (pellet). Subsequently, for preparation of the "ER-enriched fraction" the 3,500xg supernatant was centrifuged at 11.000xg, 4°C for 20 min. The mouse heart muscle mitochondria were prepared according to the procedure for "mitochondria-enriched" fraction as described (5).

### (H) Electron transport chain (ETC) and electron flux

Steady-state activity of ETC enzymatic complexes was determined as described (5, 6) using a computer-tuneable spectrophotometer (Spectramax Plus Microplate Reader, Molecular Devices; Sunny Vale, CA, USA) operating in the dual wavelength mode; samples were analyzed in temperature-controlled 96-well plates in a final volume of 300 µL. The addition of standard respiratory chain inhibitors was used to ascertain the specificity of the enzymatic assays. All enzymatic activities were normalized to protein concentration. For stimulated T cells ETC enzyme activities were measured after snap-freezing and digitonin-permeabilization (as described previously). To study electron flow from complex I or complex II to complex III digitonin-permeabilized cells or mitochondrial fractions were incubated with NADH or succinate and the reduction of cytochrome c in the presence of NaCN was measured.

### (I) Activity measurement of glycolytic enzymes

All enzymes applied were purchased from Sigma-Aldrich and prepared from rabbit muscle.
- Hexokinase (HK) activity was assayed as NADP reduction in ETC buffer containing 1 mM ATP, 1 mM glucose, 0.5 mM NADP, 0.05 U/ml glucose 6-phosphate dehydrogenase.
- Glyceraldehyde-phosphate dehydrogenase (GAPDH) activity was detected as NAD reduction in a buffer containing 10 mM KPi, 1 mM NAD, 3.3 mM cysteine 0.2 mM EDTA, 0.2 mM MgCl₂, 1 mM ADP.
- Glycerol-3-phosphate-dehydrogenase (GPD2) activity was measured as cytochrome c reduction in ETC buffer containing 5 mM glycerol 3-phosphate, 2 mM NaCN, and 60 µM cytochrome c.
- Trioso-phosphate isomerase (TPI) activity was assayed as NADH oxidation in ETC buffer containing 0.2 mM NADH, 4.9 mM DL-glyceraldehyde 3-phosphate, 0.4 U/ml α-glycerophosphate dehydrogenase (GPD1).
- Phosphofructokinase (PFK) activity was detected as NADH oxidation in ETC buffer containing 4 mM fructose 6-phosphate, 2 mM ATP, 0.5 mM NADH, 0.2 U/ml aldolase, 0.8 U/ml TPI, 0.1 U/ml GPD1.
- Pyruvate kinase (PK) activity was detected as NADH oxidation in ETC buffer containing 1 mM phosphoenolpyruvate, 1 mM ADP, 0.5 mM NADH, 2 U/ml LDH.
- Lactate dehydrogenase (LDH) activity was recorded as NADH oxidation in ETC buffer containing 1mM pyruvate, 0.5 mM NADH.
- Enolase (ENO) forward reaction was measured as NADH oxidation in ETC buffer containing 1 mM 2-phosphoglycerate, 1 mM ADP, 0.5 mM NADH, 2 U/ml LDH, 2 U/ml PK. The reverse reaction was assayed in ETC buffer containing 1 mM phosphoenolpyruvate, 1 mM ATP, 0.5 mM NADH, 2 U/ml LDH, 2 U/ml PK.
- Phosphoglycerate mutase (PGM) activity was recorded as NADH oxidation in ETC buffer containing 1 mM 2-phosphoglycerate, 1 mM ATP, 1 mM phosphoenolpyruvate, 0.5 mM NADH, 2 U/ml LDH, 2 U/ml PK. The reverse reaction was assayed in ETC buffer containing 1 mM 3-phosphoglycerate, 1 mM ADP, 0.5 mM NADH, 2 U/ml LDH, 2 U/ml PK.
- Phosphoglycerate kinase (PGK) reverse reaction was assayed in ETC buffer containing 1 mM 3-phosphoglycerate, 1 mM ATP, 0.5 mM NADH, 2 U/ml LDH, 2 U/ml PK.

### (J) Fructose-6-phosphate flux

Fructose-6-phosphate flux *via* GPD pathway was measured as NADH oxidation in ETC buffer containing 1 mM fructose 6-phosphate, 1 mM ATP, 0.5 mM NADH, 0.1 U/ml GPD1. The flux through the GAPDH branch was recorded in ETC buffer containing 1mM KPi, 1 mM fructose 6-phosphate, 1mM ATP, 0.5 mM NAD, 0.1 U/ml GAPDH.

### (K) Glucose uptake

Upon treatment, cells (1-2x10⁷ cells) were washed and suspended in Krebs-Ringer buffer. Subsequently, cells were incubated with 1 µCi/ml D-[3-³H] glucose for 10 min at 37°C. Afterwards, cells were washed twice with ice-cold Krebs-Ringer buffer and intracellular D-[3-³H] glucose was detected using a scintillation counter (LS 6500 Liquid Scintillation Counter Beckman Coulter; Brea, CA, USA).

### (L) Oxygen electrode

Mitochondrial respiratory rate was measured according to a previously described protocol (7) using computer-supported high-resolution Oroboros 1 oxygraph system (Paar, Graz, Austria). *In vitro* expanded T cells (5-10x10⁷ cells) were placed in each electrode chamber and activated with soluble anti-CD3 antibody (10 µg/m, cross-linked with GAM, 2 µg/ml) or PMA (10 ng/ml). The experimental set-up was as previously described (8). "Mitochondrial respiratory rate" was calculated by subtracting "Background oxygen consumption rate" (recorded with a reference electrode in a presence of 5 µM oligomycin) from "Total oxygen consumption rate" (as described in (8)).

### (M) Lactate quantitation

At least 2x10⁷ expanded T cells were suspended in fresh RPMI 1640 medium (+ supplements and IL-2), then activated with soluble anti-CD3 antibody (10 µg/ml) cross-linked with GAM (2 µg/ml) for 1 h. Lactate concentrations in media were detected using an Olympus AU400 system (Olympus, Tokyo, Japan) and normalized to protein concentration of respective cellular lysates.

### (N) ADP-dependent glucokinase (ADPGK) activity assay

ADPGK activity was assayed as NADP reduction in ETC buffer containing 1 mM ADP, 1 mM glucose, 0.5 mM NADP, 5 µM diadenosine pentaphosphate, and 0.05 U/ml glucose 6-phosphate dehydrogenase at pH 6.0 and 37 °C or 42 °C. Assay was essentially based on the one previously described for the recombinant mouse ADPGK protein (9).

### (O) Adenylate kinase (AK) activity assay

AK activity was assayed as NADP reduction in ETC buffer containing 1 mM ADP, 1 mM glucose, 0.5 mM NADP, 1 U/mL HK and 0.05 U/ml glucose 6-phosphate dehydrogenase at pH 7.5 and 37 °C.

### (P) ATP content determination

ATP concentration was determined using a "CellTiter Glo" assay (Promega) according to the manufacturer's instructions and 96-well luminometer (Orion L, Berthold; Bad Wildbad, Germany). Results were normalized to the protein concentration.

### (Q) Ubiquinol/ubiquinone content determination

At least 5x10⁷ *in vitro* expanded human T cells were treated with plate-bound anti-CD3 antibody (30 µg/ml) or PMA (10 ng/ml) for 1 h. Cells were washed, pelleted, snap-frozen in liquid nitrogen and placed on dry ice. Ubiquinol/-on extraction was performed by addition of ice-cold 200 µl hexan : isopropanol (2:1) and subsequent centrifugation (15.000g) for 5 min at 0 °C. Next, 150 µl of the supernatant was immediately injected and separated by RP-HPLC. HPLC separation and identification conditions were based on previously published methods (10, 11). Chromatographic system applied: LaChrom LC-7100 low pressure gradient pump with proportioning valves, L-7350 column thermostat with L-7350/L-7351 Peltier cooling module, L-7450A UV/VIS-DAD - diode-array detector (Merck-Hitachi; Darmstadt, Germany), pulsed amperometric detector (PAD) 817 Bioscan (Metrohm; Herisau, Switzerland), column LiChrospher RP18e 125x4 mm (Merck; Darmstadt, Germany), 4-channel degasser (Knauer; Berlin, Germany), isocratic elution with methanol : hexan : isopropanol : acetic acid (83 : 27.5 : 1.5 : 1.5 v/v + sodium acetate 4,2 g/l; pH=6), flow rate - 1 ml/min, temp. 25°C. Identification was based on UV/VIS-DAD absorption spectra and facilitated by redox response detected with volt-amperometric detector. Quantitation was performed based on UV-VIS chromatograms at wavelengths: 275 nm (ubiquinone) and 289 nm (ubiquinol). Selected qualitative and quantitative results of UV-VIS/DAD analysis were confirmed by HPLC-MS/ESI (Agilent; Santa Clara, CA, USA).

### (R) Luciferase reporter assay for NF-κB activation

J16-145 Jurkat T cells stably transfected with *Gaussia* luciferase-based NF-κB reporter were kindly provided by Dr. Marcus Brechman and Dr. Rüdiger Arnold. Briefly, after exchanging medium cells were induced with Iono (10 µM) and/or PMA (10 ng/ml) for 6 h in triplicates for each experimental condition. 20 µl of collected medium was applied to luminescence read-out using "Gaussia-Juice" (P.J.K.; Kleinblittersdorf , Germany) and 96-well plate luminometer (Orion L; Berthold, Bad Wildbad, Germany). Luminescence signal was normalized to the number of living cells assessed by Trypan-Blue exclusion method.

### (S) Immunoprecipitation and WB

At least 8x10⁷ F-ADPGK or EV Jurkat T cells were lysed in a buffer containing 150mM NaCl, 5mM EDTA, 10mM Tris-HCl, 0.5% Triton X-100 for 45 min at 4°C. Lysats were centrifuged at 11.000g, 4°C for 10 min. Supernatants were applied onto agarose beads covalently cross-linked with anti-FLAG M2 antibodies (ANTI-FLAG® M2 Affinity Gel, Sigma). Washing steps and elution with FLAG peptide were performed according to manufacturer's protocol. Eluats were directly subjected to ADPGK activity assay or frozen and analyzed by WB (3).

### (T) ADPGK over-expression experiments

For transient expression of WT-ADPGK cDNA sequence of human ADPGK transcript variant 1 (UniProtKB canonical sequence Q9BRR6-1) cloned into pCMV6-AC plasmid (provided by Origene; Rockville, MD, USA) or empty control pCMV6-AC vector (Origene) were used. J16-145 cells or J16-145 NF-κB *Gaussia* luciferase reporter clone were transfected with 2 µg plasmid DNA/transfection using AMAXA nucleofection technology ("Cell Line Nucelofector® V kit", Lonza; Cologne, Germany) according to the manufacturer's instructions. 24 h post-transfection cells were subjected to the experimental procedures. Pools of J16-145 cells stably expressing FLAG-ADPGK protein (F-ADPGK Jurkat cells) or empty-vector (control cells) were generated using retroviral expression vectors provided by Addgene (Cambridge, MA, USA): pWZL-Neo-Myr-Flag-ADPGK (Addgene plasmid 20417, N-terminal FLAG-tag on a protein backbone, UniProtKB Q9BRR6-1) or pWZL-Neo-Myr-Flag-DEST (Addgene plasmid 15300), respectively (12). Retroviral vectors were transfected by calcium phosphate method into Phenix-Ampfo producing cells (Allele Biotechnology; San Diego, CA, USA). Viral transduction was performed according to manufacturer's protocol using polybrene and spin-infection (2 h / 2.000g). Cells were cultured under a selection pressure in RPMI 1640 (+ L-glutamine), 10% FCS supplemented with 1 mg/ml G418 sulphate (Roth; Karlsruhe, Germany).

### (U) Real time quantitative reverse transcription PCR (real time qRT-PCR)

RNA was isolated with TRIzol reagent (Invitrogen) (CLL cells and control B cells) or "RNeasy Mini" kit (QIAGEN; Hilden, Germany) (all other samples) according to the manufacturer's instructions. Total RNA (1 µg) was reverse-transcribed with a "Reverse Transcription (RT)-PCR kit" (Applied Biosystems; Carlsbad, CA, USA). Quantitative real-time-PCR was performed using the "Power SYBR Green PCR Master Mix" (Applied Biosystems). Gene expression was analyzed using the 7500 Real-Time PCR Systems and Sequence Detection Software, Applied Biosystems, v. 2.0.2. Gene expression levels were normalized using β-actin expression as an endogenous reference. Induction ratios (*X*) were calculated using the formula *X* = 2^{-ΔΔ*C*t}, where Ct stands for cycle threshold and Δ*C*t = *C*t _{gene of interest} - *C*t _{reference gene}· ΔΔ*C*t is the difference between the Δ*C*t values of the "induced" samples and the Δ*C*t of the corresponding "non-induced" sample. The mean induction ratios were calculated. The relative ADPGK basal expression levels in B cell samples from CLL patients and healthy donors were compared using factor *Y* = 2^{(*C*t gene of interest - *C*t GAPDH)} × 1000 (*1*). To correct for inter-experimental variation the values obtained from two separate experiments (two separate 96-well plates with triplicated 5 CLL samples and 4 or 5 healthy donor samples each) were subjected to z-transformation. The following primers were used for gene expression analysis: β-actin, sense 5-ACCGTGAGAAGATGACCCAGA-3', anti-sense 5'-TCACCGGAGTCCATCACGAT-3'; IL-2, sense 5'-CAACTGGAGCATTTACTGCTG-3', anti-sense 5'-TCAGTTCTGTGGCCTTCTTGG-3'; IL-8, sense 5'-GAATGGGTTTGCTAGAATGTGATA- 3', anti-sense 5'-CAGACTAGGGTTGCCAGATTTAAC- 3', ADPGK, sense 5'-TCATTGCAGGAAGTGGATGA- 3', anti-sense 5'-GCATGGGGAGCTTTTAACTG-3', IκBα, sense 5'-GTCAAGGAGCTGCAGGAGAT-3', anti-sense 5'-GATGGCCAAGTGCAGGAA- 3', GPD2, sense 5'-ACCCTGGCTGGAGGAACT3', anti-sense, 5'-CCCTTTCACTGCCTTTTGAA3' and IL-4 primers as published elsewhere (1).

### (V) siRNA transfection and knock-down

siRNA oligonucleotides used for transfection were as follows: non-silencing control (unlabeled "AllStars" non-silencing, validated siRNA, QIAGEN), specific for human ADPGK (Hs_ADPGK_5-8 FlexiTube siRNA, QIAGEN) or human GPD2 (Hs_GPD2_1, 4-6 FlexiTube siRNA, QIAGEN). Jurkat T cells or peripheral "resting" human T cells were transfected by nucleofection ("Cell Line Nucelofector® V kit" or "T Cell Nulcleofector® kit", Lonza) performed with 800 nM of siRNA oligonucleotides according to the manufacturer's instructions.

### (W) Blue-native polyacrylamide gel electrophoresis (BN-PAGE) and WB

BN-PAGE analysis of mitochondria-enriched fractions was performed according to Haeger *et al.* (13) with modifications. Protein complexes of the mitochondrial membrane were solubilized in the presence of 1.5 M 6-aminocaproic acid, 50 mM Bis-Tris and digitonin (6 g/g protein) at 4°C for 30 min. Subsequently, samples were centrifuged at 11.000xg and 4°C for 30 min. The supernatant was supplemented with sample buffer (1.5 M 6-aminocaproic acid, 50% glycerol, 1% (w/v) Coomassie blue G250) and immediately applied on a BN-Gel (3-13%). The gel was run for 16 h at a constant current of 35 V. Proteins were blotted for 2 h with a current limited to 0.8 mA/cm² membrane. Bovine heart-purified complex III (BC1 complex) was a kind gift from Prof. Ulrich Brand.

### Example 2

### TCR triggering induces a Warburg effect-like metabolic shift in pre-activated T cells

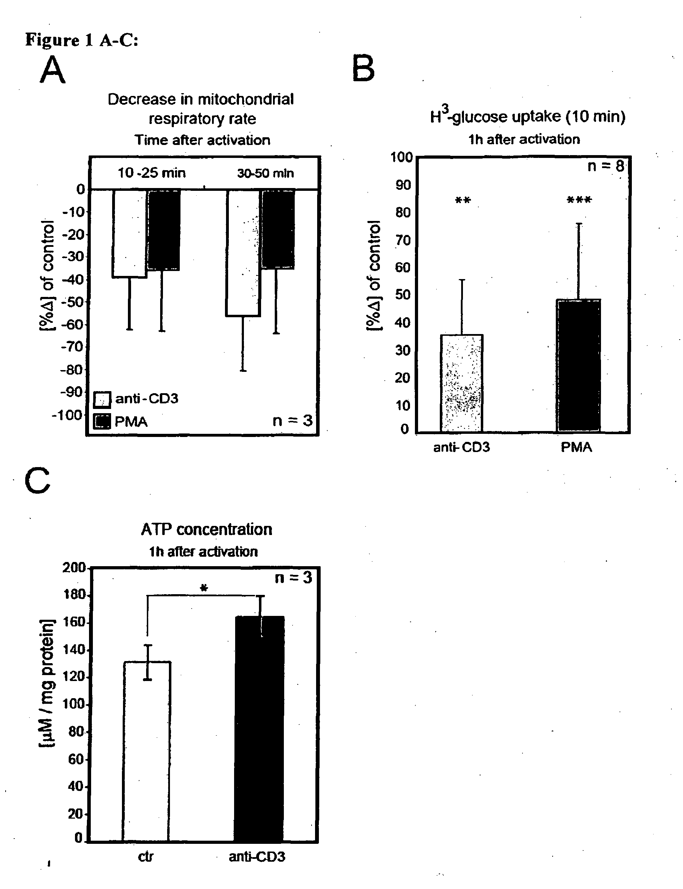

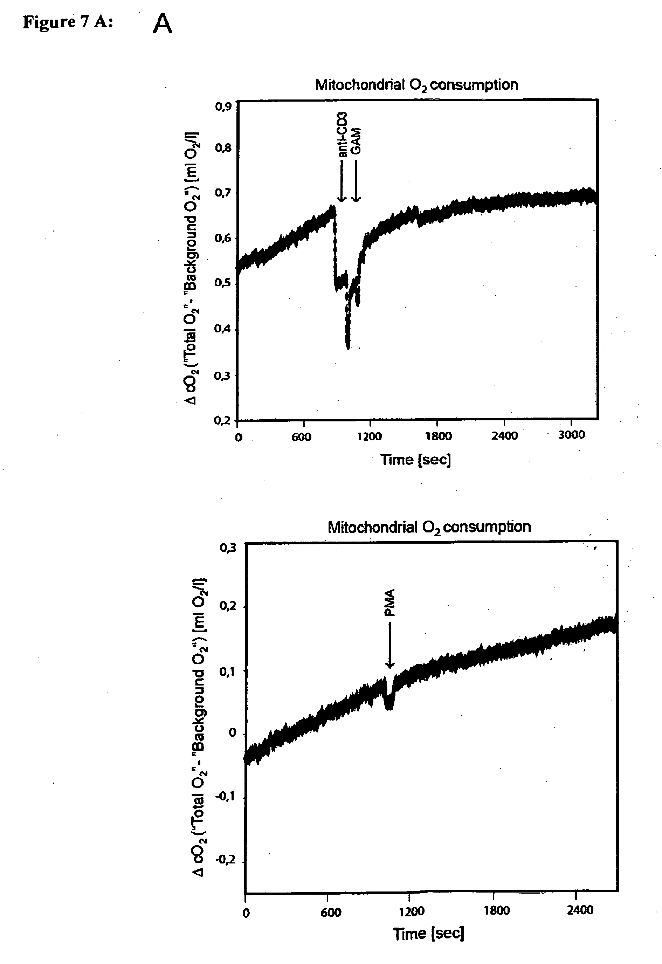

A reduction of mitochondrial respiration and an increase in glycolysis are central to the Warburg effect (11-14). An oxygen electrode was applied to measure activation-induced changes in mitochondrial respiration of intact human T cells. In line with the Warburg phenotype, treatment with agonistic anti-CD3 antibody or PMA resulted in a significant inhibition of mitochondrial oxygen consumption (Figure 1A and Figure 7A), whereas the cellular uptake of radioactively labeled glucose rapidly rose after stimulation (Figure 1B). The consecutive rise in intracellular ATP levels (Figure 1C) highlighted that in spite of normoxia, TCR triggering of *in vitro* expanded human peripheral T cells led to a rapid shift towards an even more glycolytic phenotype.

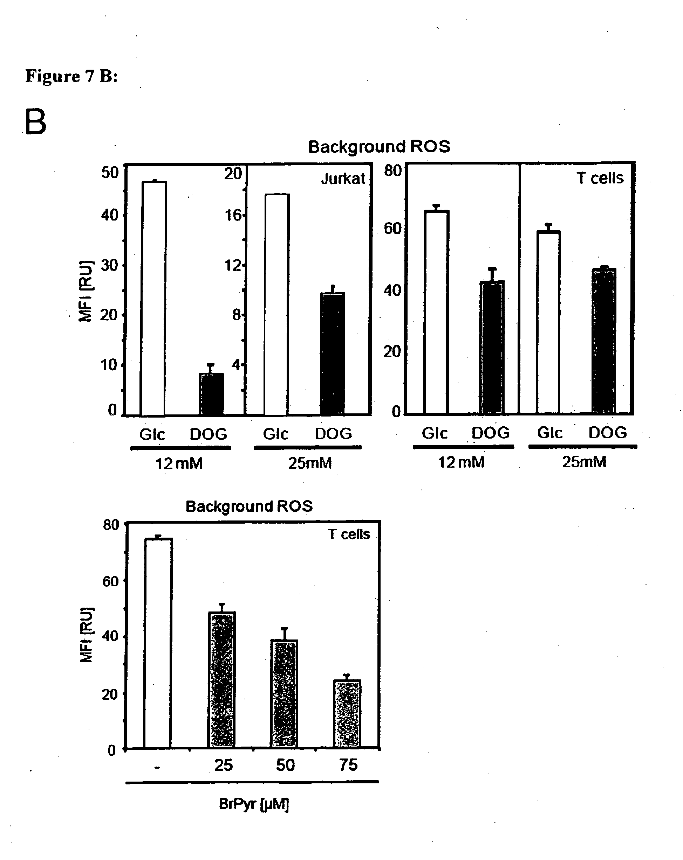

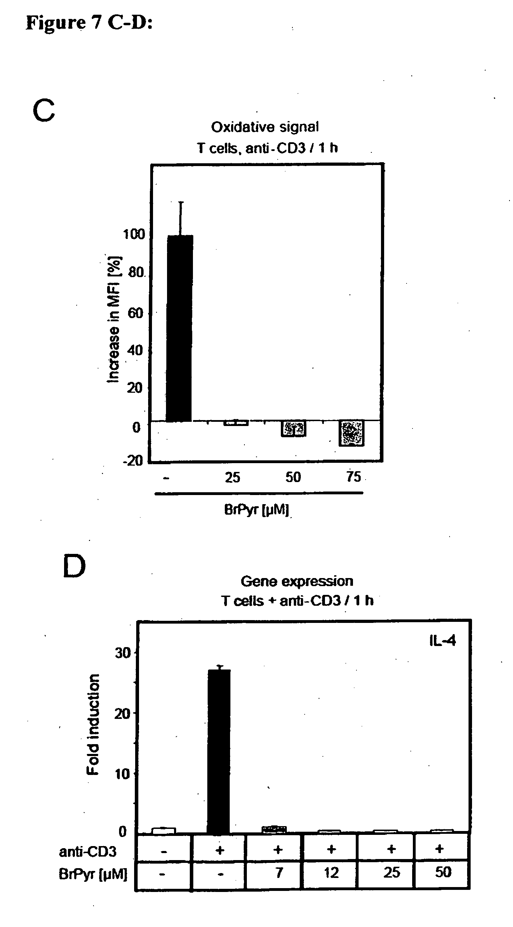

To verify a causative link between observed metabolic changes and enhanced mitochondrial ROS production, the influence of 2-deoxy-glucose (DOG) and 3-bromopyruvate (BrPyr), blockers of glucose metabolism, on the generation of activation-induced ROS was tested. After brief pre-incubation (30 min) with DOG/pyruvate expanded human T cells and Jurkat T cells generated lower amounts of activation-induced ROS as compared to cells pre-incubated with equimolar concentrations of glucose (Glc)/pyruvate (Figure 1D). Treatment with the more potent inhibitor, BrPyr, resulted in complete inhibition of TCR-induced mitochondrial ROS generation and activation-induced NF-κB-dependent IL-2, IL-4 and IκBα gene expression (Figure 1E and F, Figure 7C and D). Moreover, both agents reduced the intracellular oxidative background (Figure 7B). Thus, T cell activation-induced mitochondrial ROS generation is associated with a rapid metabolic shift towards glycolysis, commonly defined as Warburg effect.

### Example 3

### Generation of the oxidative signal is accompanied by major bioenergetic and ultrastructural changes within the mitochondria

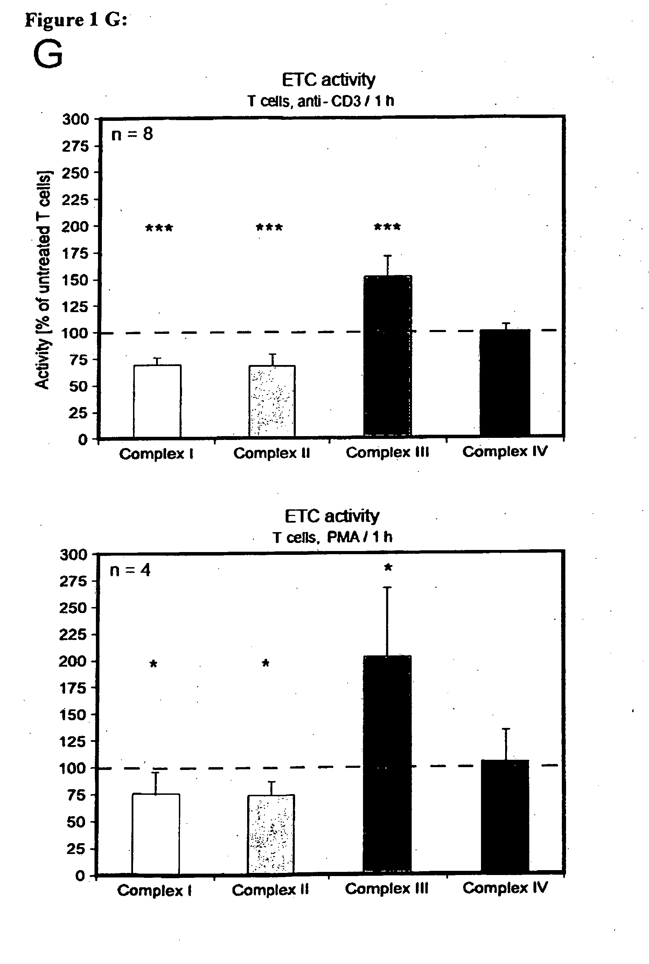

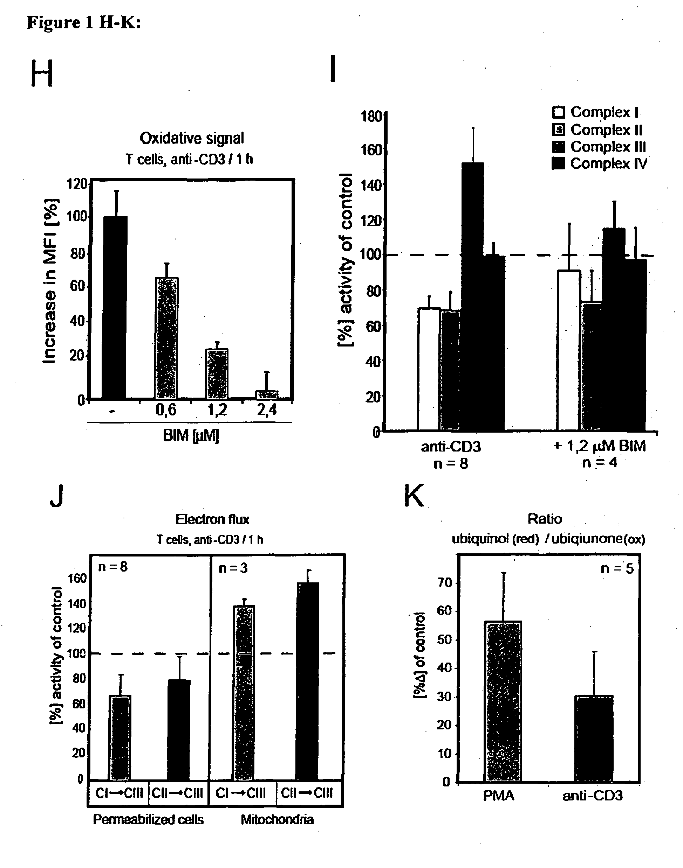

TCR-triggered PKCθ induces oxidative signal generation by mitochondrial respiratory complex I (*3*). To gain more insight into the relationship between the activation-induced Warburg metabolic shift and the mechanism of mitochondrial ROS release the bioenergetic status of respiratory chain complexes was investigated. To this end, snap-frozen and digitonin-permeabilized *in vitro* expanded human T cells were applied. As shown in Figure 1G (upper panel), TCR triggering resulted in a significant decrease of enzymatic activities of complexes I and II, while activity of complex III was increased. A similar pattern could be observed in cells treated with PMA (Figure 1G, lower panel). The data show that these phenomena are independent from TCR-triggered mitochondrial Ca²⁺ uptake. Moreover, they suggest that a DAG/PKCθ-dependent pathway is involved. In line with this assumption, pre-treatment of T cells with bis-indolyl-maleimidate I (BIM), an inhibitor of PKC and TCR-induced ROS generation, significantly blocked the observed changes (Figure 1H and I).

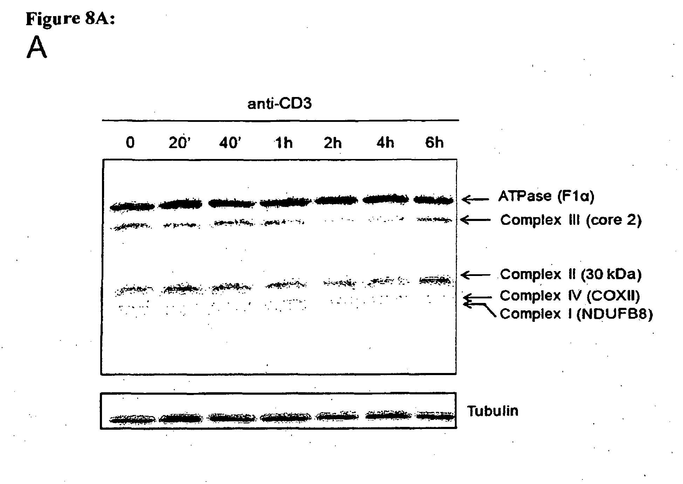

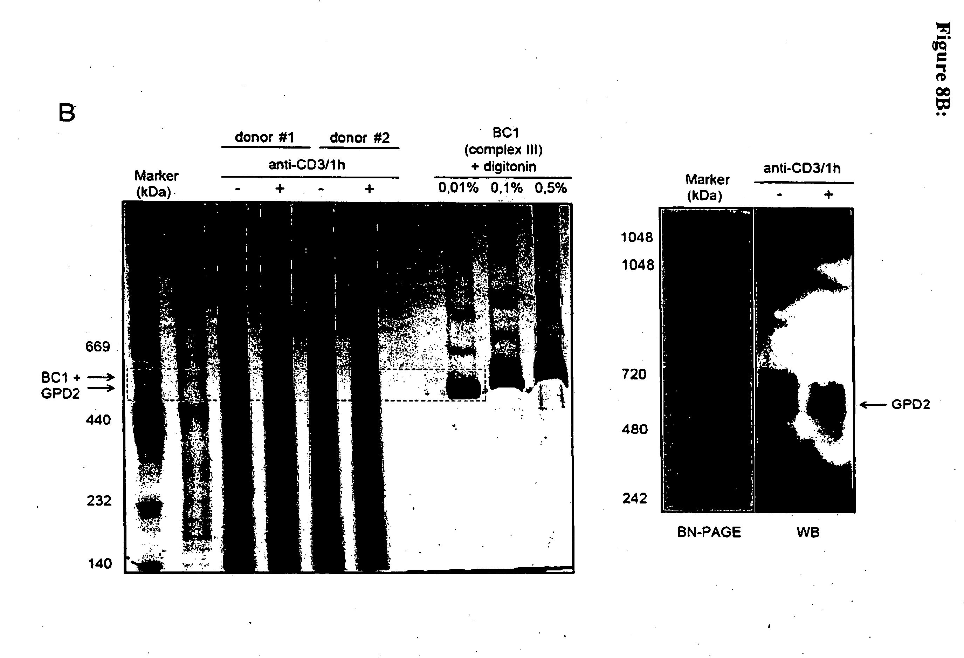

Furthermore, the observed differential pattern of enzymatic activities did not correspond with changes in the protein content of the respective complexes (Figure 8A) and the migration in Blue Native (BN)-PAGE gels (Figure 8B). Interestingly, the ultrastructure of mitochondria changed upon TCR or PMA triggering (Figure 2). Disarrangement and distortion of the cristae were particularly pronounced in mitochondria of TCR-activated cells (Figure 2C), while PMA-treatment led to various degrees of alterations (Figure 2B). No rupture of outer and inner mitochondrial membranes could be detected. In addition, since these changes were observed rapidly (1 h) and dependent on the activation phenotype of cells (*e.g.* rise in ATP level, up-regulation of glucose transport, and chromatin relaxation), their apoptotic origin was excluded. The observed alterations closely resembled those typical for cells of highly glycolytic tumors and were previously reported to occur as an adaptive response to hypoxia (*24*) or transient mitochondrial metabolic adaptation to state IV of respiration (rate-limiting low ADP content, high ROS production) (*25*). Thus, the mitochondrial electron micrographs indicate a low respiratory activity, which parallels the enhanced glycolytic phenotype of activated T cells.

To better understand these phenomena, electron flow rates from complex I/II to complex III using either permeabilized cells or isolated mitochondria (Figure 1J) were analyzed. In the first case, T cell activation led to a decreased electron flow rate between complex I or II and complex III, in line with the results presented in Figure 1G. Mitochondrial isolation abolished these effects and resulted in an enhanced electron flow towards complex III upon T cell activation (Figure 1J). These results suggest a transient character of the activation-induced decrease of complex I/II activities due to the involvement of a labile agent. The fact that enhanced complex III activity is not impaired by the mitochondrial isolation procedure implies a stable modification of complex III.

The observed bioenergetic changes could be attributed to the hyper-reduced state of ubiquinone, the electron carrier between complexes I/II and complex III (*26,27*)*.* Accumulation of reduced ubiquinone (ubiquinol) is likely to decrease the activities of complex I and II in intact cells by blocking the electron flow. This effect would be lost after isolation of mitochondria due to the oxidation of ubiquinol (Figure 1J). Moreover, the reverse electron transfer (RET), a major mechanism of mitochondrial ROS generation *via* complex I, is mediated by a highly reduced pool of ubiquinone (*26,27*). Ubiquinol accumulation is also crucial for hyperglycemia/hypoxia-induced mitochondrial ROS release (*18-21*).

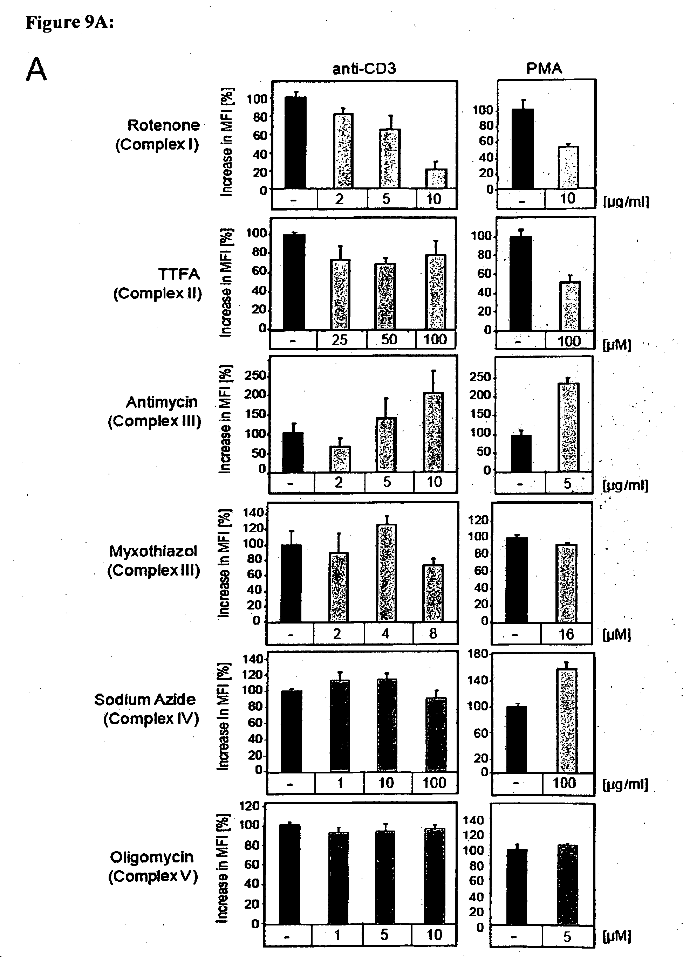

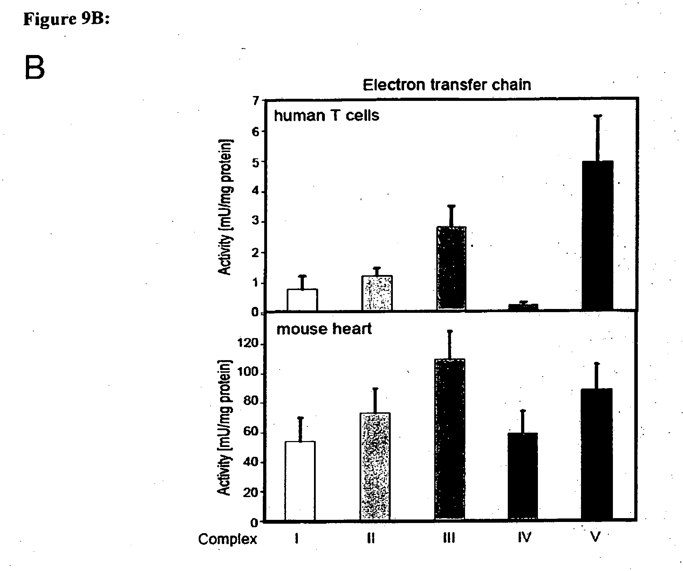

To gain a better insight into the ubiquinone redox status upon T cell activation, the influence of a panel of respiratory chain inhibitors on oxidative signal generation was tested. According to the literature, the observed inhibition by complex I blockers, rotenone and metformin (*1, 3*), as well as moderate inhibition by the complex II blocker, TTFA, suggest a RET-based mechanism of activation-induced mitochondrial ROS release (Figure 9A) (*26*). The ROS signal was not blocked by NaN₃, a complex IV inhibitor, and oligomycin, an ATP synthase inhibitor (Figure 9A). Strikingly, T cell activation-induced mitochondrial ROS generation was increased by antimycin, a complex III inhibitor binding to the matrix site, but not by myxothiazol, an inhibitor interacting with the intermembrane space part of complex III (Figure 9A). These results suggest an importance of intra-complex III ubiquinone cycling as reported for hypoxia-induced mitochondrial ROS release (*20*). Noteworthy, in human T cells, the detected endogenous complex IV activity is about 8x lower than complex III activity (Figure 9B) forming a bottleneck for the regeneration of ubiquinone. Thus, changes of the ubiquinone redox status upon T cell activation was investigated. HPLC-based analysis of the extracts from snap-frozen activated T cells revealed a significant rise in the content of ubiquinol over ubiquinone, reaching 30% upon TCR- and 57% upon PMA-mediated triggering (Figure 1K). The obtained data demonstrate a hyper-reduced status of the ubiquinone pool upon T cell activation and strongly suggest a causative role of RET in the generation of the oxidative signal.

### Exsample 4

### Diverted glycolytic flow leads to a GPD2-mediated mitochondrial ROS release

Mitochondrial ROS production induced by hyper-reduced ubiquinone is known to be associated with an increased glucose metabolism in hyperglycemic or hypoxic cells (*18*-*21*). To assay the glycolytic metabolic flow immediately after TCR triggering activities of all major glycolytic enzymes were measured (Figure 3A and B). Interestingly, the only enzymatic activity significantly changed after TCR stimulation was the reverse reaction of enolase (ENO) (Figure 3B). Since the assay used only detects a phosphoenolpyruvate (PEP)-specific ATP hydrolysis via monitoring the reduction of pyruvate in the presence of NADH, inhibitors were used to further characterize the detected enzymatic activity. NaF, an ENO inhibitor, blocked the detected reverse ENO activity. Interestingly NaVO₃, an inhibitor of histidine phosphorylation (*28*), reduced the activity but did not block it. Foremost, it abolished its TCR-induced increase (data not shown). Noteworthy, phosphoglycerate mutase (PGM) activation mediated by PEP-dependent histidine phosphorylation has been recently described in fast proliferating normal and cancer cells, that express the pyruvate kinase (PK) isoform M2 (PK-M2) (*29, 30*). Since PK-M2 is highly expressed in activated T cells (*31*), the detected rise in the reverse ENO-like activity could be mediated by the recently described unknown PEP-utilizing histidine kinase (*29*). This is further supported by the finding that the observed ENO-like activity was only partially ATP-dependent, and its activation-induced increase ATP-independent.

It was suggested that PGM histidine phosphorylation reverses the flux through the lower glycolytic pathway initiated by GAPDH (*29*), and thus, could redirect it to the alternative flux through the cytosolic glycerol-3-phosphate dehydrogenase (GPD1/2) pathway. Indeed, TCR triggering clearly enhanced fructose-6-phosphate metabolism in the direction of GPD1, while GAPDH-mediated fructose-6-phosphate turn-over was slightly decreased (Figure 3C). In addition, production of lactate, the end-product of the GAPDH pathway, was decreased despite the increased glucose uptake (Figure 3D and 1B). Thus, we investigated the activity status of GPD2, the mitochondrial isoform of GPD. GPD2 activity was significantly up-regulated upon TCR triggering (Figure 3E). PKC inhibition by BIM significantly blocked TCR-induced GPD2 activation (Figure 3E). Moreover, in line with the previous report (*32*), PMA treatment also moderately up-regulated GPD2 activity in mitochondria-enriched membrane fractions of pre-activated human T cells (Figure 3E). Since GPD2 is a Ca²⁺ binding enzyme, the anti-CD3 triggering-induced rise in intracellular Ca²⁺ concentration converges with reported PMA (DAG)-dependent activation (*32*, *33*).

GPD2 transfers electrons to the respiratory chain *via* reduction of ubiquinone. Therefore, up-regulation of its enzymatic activity could result in a RET-mediated ROS release due to hyper-reduced ubiquinone (*26,27, 34-36*). To investigate this possibility, the effects of down-regulation of GPD2 expression on activation-dependent oxidative signal generation, NF-κB triggering and gene expression were analyzed. siRNA-mediated knock-down of GPD2 expression resulted in a decreased generation of the oxidative signal (Figure 3F and G). Correspondingly, decrease in GPD2 abundance inhibited PMA- or PMA/iono-induced activation of an NF-κB luciferase reporter as well as IL-2 expression in Jurkat T cells (Figure 3H and I).

Furthermore, BN-PAGE-based WB strongly suggested an association of GPD2 with respiratory complex III (Figure 8B). Direct interaction of GPD2 with respiratory chain super-complexes may partially explain the observed increase in complex III activity upon TCR triggering or PMA treatment (Figure 1G, J). In conclusion, T cell activation results in a diversion of the glycolytic flux towards GPD2, GPD2 activation, hyper-reduction of ubiquinone and RET-mediated ROS release.

### Example 5

### T cell activation-induced ADPGK enzymatic activity up-regulates glycolytic flux

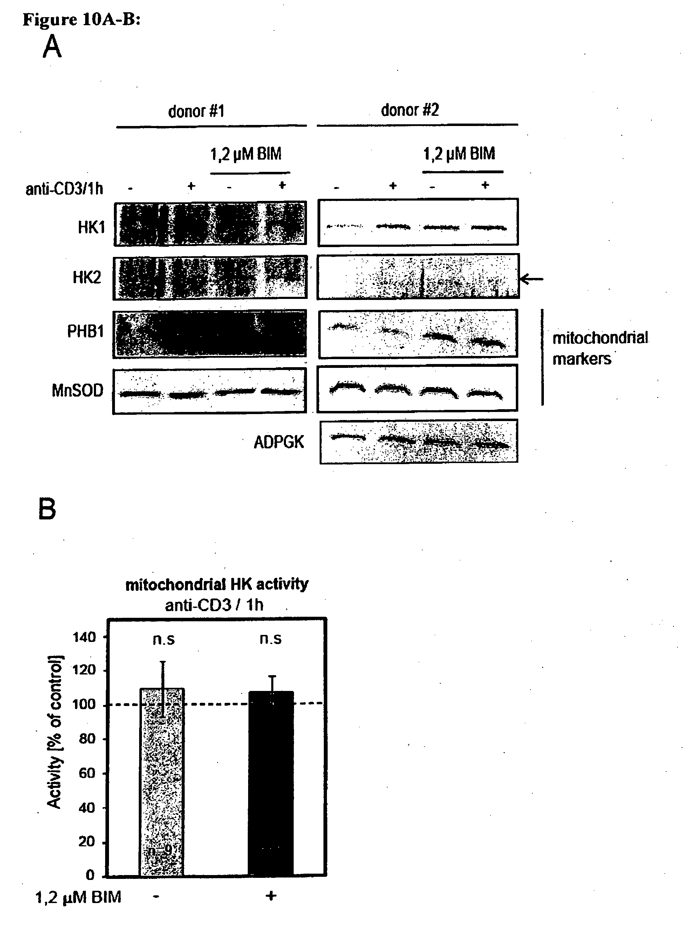

Glucose-induced hyper-reduction of ubiquinone and mitochondrial ROS release are thought to depend on up-regulated glycolytic flux (*18,19*). Glycolysis is a highly regulated sequence of reactions with major regulatory points at HK, PFK, GAPDH, and PK (Figure 3A). The results of the present experiments showed no change of any of these enzymatic activities (Figure 3B). Association of HK with mitochondrial VDAC resulting in a loss of HK product inhibition acts as another mechanism that up-regulates glucose flux (*37*). WB analysis of mitochondria-enriched membrane fractions of expanded human T cells revealed high HK1 and low HK2 levels (Figure 10A). Nevertheless, TCR triggering did not result in a significant enhancement of HK1/2 association with mitochondria or up-regulation of mitochondria-associated HK activity (Figure 10A and B). Next, mitochondria-associated HK activity coupled to respiration-mediated ATP generation was investigated (Figure 4A). TCR triggering clearly induced a HK-like activity in presence of the respiratory substrates, ADP and succinate (Figure 4B). This activity was only mildly blocked by the adenylate kinase (AK) inhibitor diadenosine pentaphosphate (Ap5A, 5 µM, up to 40% inhibition), whereas Ap5 strongly inhibited mitochondrial AK (5 µM, 80-90% inhibition). Most importantly, the activation-induced rise in HK-like activity was unaffected by Ap5A. Surprisingly, this enhanced enzymatic activity was independent from addition of succinate, but clearly dependent on the presence of ADP. Furthermore, insensitivity to the respiratory chain inhibitor NaCN and the uncoupler CCCP demonstrated its actual independence from ATP produced by mitochondria (Figure 4B). Intrigued by these results, the literature was searched and a protein able to phosphorylate glucose could be identified by utilizing ADP - the ADP-dependent glucokinase (ADPGK) (*38*). Although, typical for thermophylic *Archaea,* ADPGK exists in mammals and is highly expressed in human haematopoetic cells, including T cells (Figure 5E) (*31,39*). Furthermore, a reported lack of inhibition by glucose-6-phosphate makes ADPGK a particularly good putative activator of glycolytic flux (*38*).

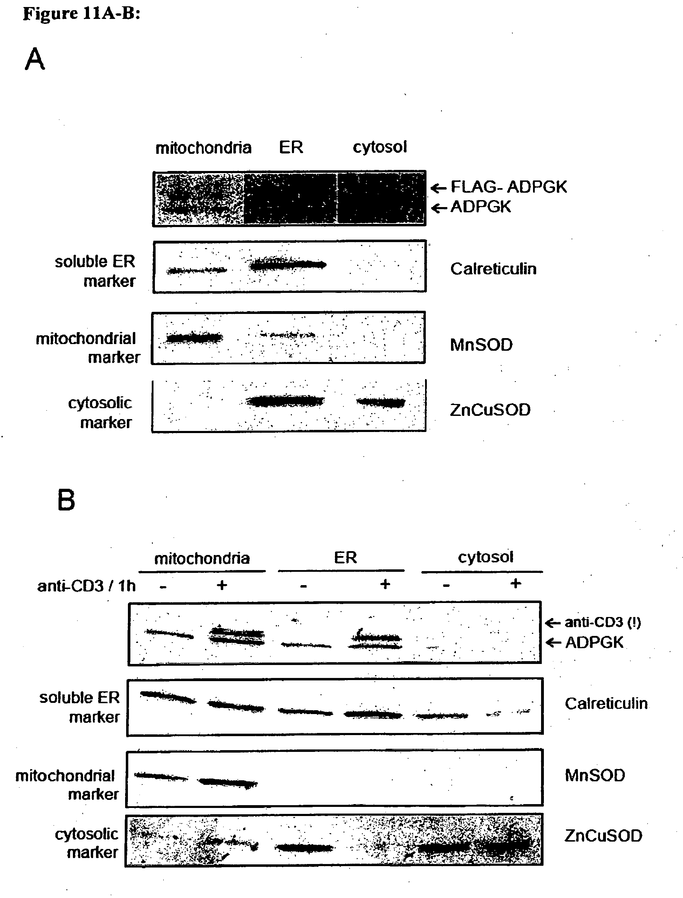

Indeed, enhanced ADPGK activity occurred in mitochondria-enriched membrane fractions ("high g" fractions) isolated from anti-CD3-treated human T cells (Figure 4C). Comparable up-regulation of enzymatic activity could be achieved by PMA treatment of human T cells or Jurkat T cells (Figure 4C), indicating an independence from Ca²⁺-mediated TCR signaling. Furthermore, T cell activation-induced enhancement of the ADPGK-like enzymatic activity was completely abolished by the PKC inhibitor BIM (Figure 4C). No up-regulation of ADPGK content upon TCR stimulation could be demonstrated (Figure 4D). Interestingly, the ADPGK sequence contains a putative signal peptide for transport into the endoplasmic reticulum (ER) (Figure 4E and Figure 14). Since T cell mitochondria are smaller than the ones in other tissue (*e.g.* liver), a 11.000xg centrifugation step was utilized to increase the yield of mitochondria in the mitochondria-enriched fractions ("high g" mitochondrial fractions). Such fractions contain also ER as seen in electron micrographs and WB (calreticulin - an ER marker) (Figure 4D). To gain more insight into the subcellular localization of ADPGK, "mitochondria-enriched" and "ER-enriched" fractions were prepared (see Example 1) clearly showing an ER-association of ADPGK protein in T cells and Jurkat T cells (Figure 11).

ADPGK activity in "ER-enriched" fractions was characterized with respect to its kinetic parameters. The enzyme revealed a striking pH optimum of 6.0 and a Km for glucose of 0.086 mM (pH 6.0, 37°C). Obtained values resemble the ones reported for mouse recombinant ADPGK (*38*), and are in a range similar to so-called low-Km HKs 1-3. Furthermore, ADPGK was substrate-inhibited at glucose concentrations higher than 5 mM.

Next, Jurkat T cells stably expressing ADPGK protein with an N-terminal FLAG tag were generated (F-ADPGK Jurkat cells, Figure 4E). As for wild-type ADPGK protein (WT-ADPGK), the FLAG-ADPGK protein (F-ADPGK) was found in the ER-enriched and mitochondria-enriched but not in cytosolic fractions (Figure 11A and Figure 4D). Cells expressing F-ADPGK showed a higher basal as well as an enhanced PMA-induced ADPGK-like activity as compared to control cells (Figure 5E, lower panel). Immunoprecipitated FLAG-ADPGK protein demonstrated ADPGK activity, which could not be measured in precipitates from control lysates (Figure 4F, lower panel). The results were confirmed by WB (Figure 4F, upper panel). Thus, for the first time, a fully active human ADPGK was shown. In conclusion, it was found that TCR triggering up-regulates glycolytic flux by activating ADPGK in a PMA(DAG)- and PKC-dependent manner.

### Example 6

### ADPGK mediates generation of the oxidative signal

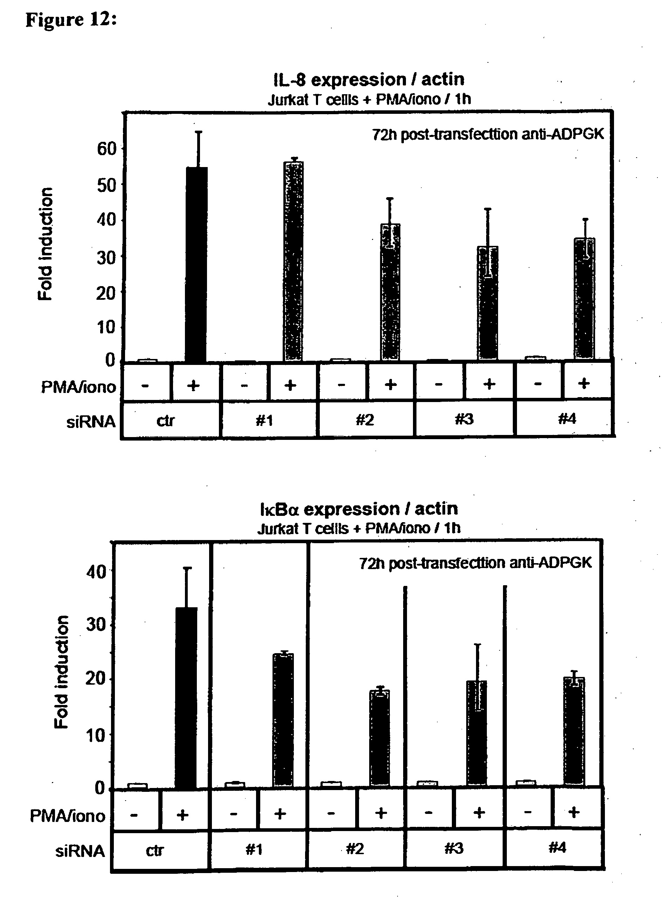

Next, the influence of lowering ADPGK expression on activation-induced ROS generation was assayed. Decreased expression of ADPGK (Figure 5A) inhibited PMA-triggered production of ROS in Jurkat T cells (Figure 5B). Concomitantly, knock-down of ADPGK resulted in inhibition of PMA- or PMA/iono-triggered NF-κB activation (Figure 5C) and NF-κB-dependent expression of IL-2, IκBα and IL-8 genes (Figure 5D and Figure 12). "Resting", non-expanded T cells also generate the oxidative signal from mitochondria (*1*). Likewise, activation of "resting" T cells led to a rise in GPD2 and ADPGK enzymatic activities (Figure 5F). Low induction of the enzymatic activities correspond to a lower extent of ROS generation in "resting" T cells as compared to *in vitro* expanded "pre-activated" T cells (*1*). Nevertheless, siRNA-mediated knock-down of ADPGK expression in "resting" peripheral human T cells also resulted in lowering of the activation-induced oxidative signal (Figure 5G).

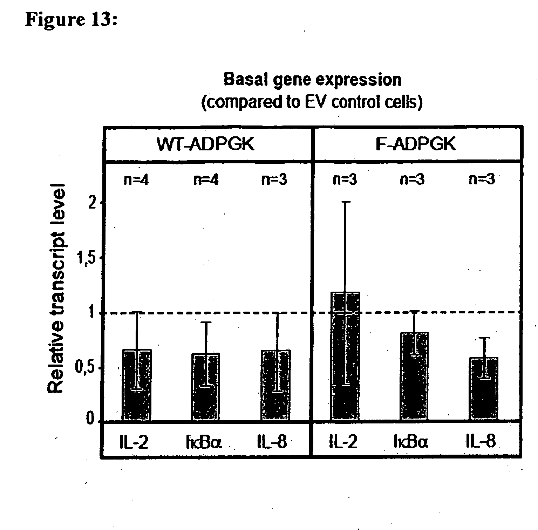

Furthermore, enhanced PMA-induced ADPGK activity in F-ADPGK Jurkat cells (Figure 4E) resulted in increased ROS production followed by potentiated induction of NF-κB-dependent genes (IL-2, IL-8 and IκBα) (Figure 6A, C). Similar results were obtained for Jurkat T cells transiently over-expressing WT-ADPGK (Figure 6B and C). In these cells activation resulted in enhanced NF-κB triggering and potentiated induction of NF-κB dependent genes as compared to empty vector (EV)-transfected control cells (Figure 6B and C). Interestingly, due to so far unknown mechanism, ADPGK overexpression tends to decrease basal transcript levels of NF-κB dependent genes (Figure 13). Taken together, the present data demonstrate a positive regulatory role of ADPGK in the process of T cell activation.

### Example 7

### ADPGK activity rises at higher pro-inflammatory temperature

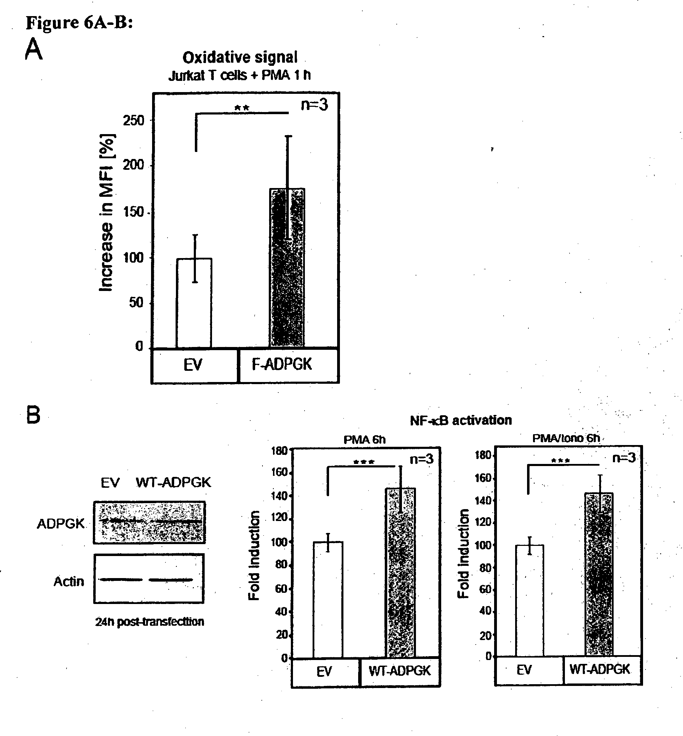

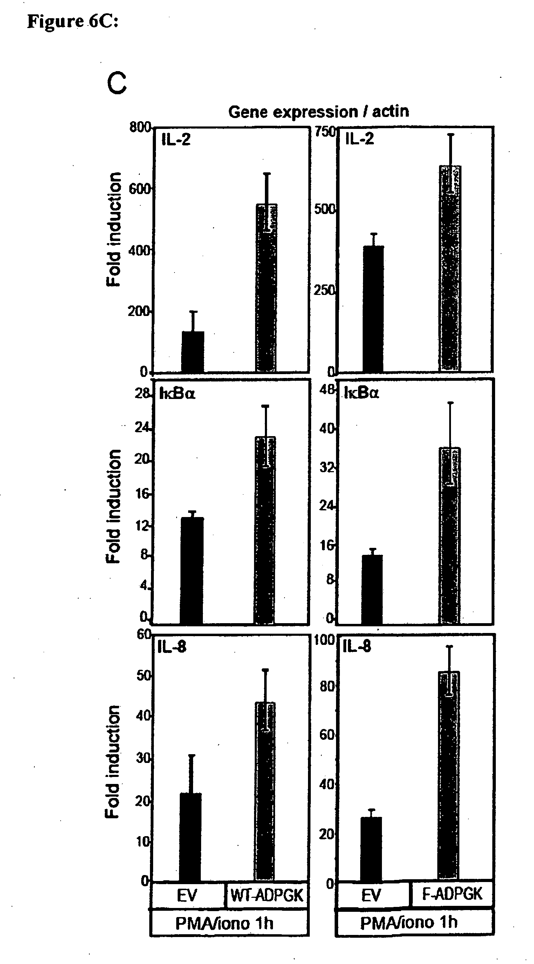

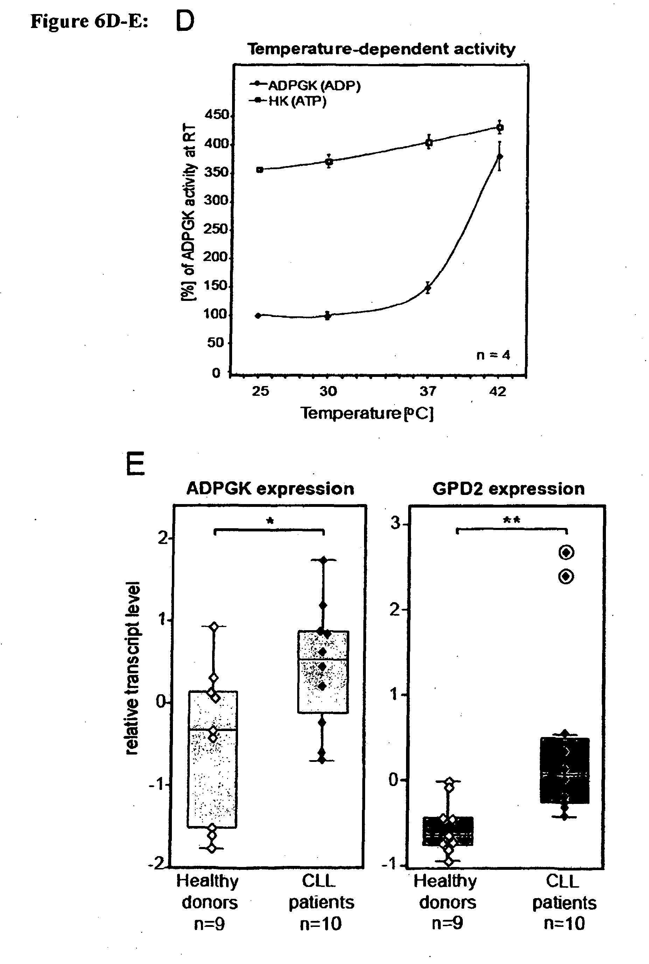

ADPGK activity was primarily described for thermophilic *Archaea (38, 40).* Notably, a secondary structure prediction for mammalian ADPGK reveals a high structural similarity to known secondary structures of thermostable archaeal ADPGK despite a low sequence homology (Figure 14) (*40,41*). Therefore, the temperature dependence of ADPGK activity weas tested in human T cells. As shown in the Figure 6D, velocity of the ADPGK-catalyzed reaction exponentially rose within the physiological temperature range and was 3.5x higher at 42°C than at 37°C. In contrast, the temperature-driven rise in HK activity remained linear. Furthermore, at 42°C ADPGK-mediated reaction reaches a velocity comparable with that of HK. These data suggest thermostability of human ADPGK and strengthen its possible role in the inflammatory response and NF-κB triggering. Thus, increased temperature at inflammatory sites might facilitate T cell activation and NF-κB-dependent expression of pro-inflammatory cytokines.

### Example 8

### ADPGK and GPD2 are highly expressed in malignant chronic lymphocytic leukemia (CLL) cells

Many cancers strictly depend on the constitutive activation of the NF-κB pathway (*16*). High intrinsic levels of ROS or Warburg phenotype are features of many malignancies (*11,14,42*). Therefore, ADPGK expression in cancer cells was investigated. T cell-originated cancers are rare in humans as compared to B cell malignancies. Particularly, CLL constitutes one of the most common leukemias with fatal prognosis (*43*). Moreover, CLL cells are endowed with enhanced NF-κB activity, glycolysis and high intrinsic ROS levels (*15, 17, 43-45*). Thus, ADPGK and GPD2 expression levels in malignant CLL cells and normal B cells were compared (Table 1).

**Table 1**

| **Additional information about patients analyzed in** **Figure 8** | | | | | |
|---|---|---|---|---|---|
| **Number** | **Age** | **Gender** | **Stage** | **Therapy** | **Additional data** |
| 028/09 | 52 | M | Binet A | no | FISH - no changes |
| 032/09 | 46 | W | Binet A | no | del 13q14 |
| 038/09 | 70 | W | Binet B | no | Trisomy 12 |
| 041/09 | 43 | M | Binet B | no | FISH - no changes |
| 047/09 | 86 | M | Binet A | no | del 13q14 |
| 063/09 | 70 | W | Binet A | no | n.d. |
| 090/09 | 34 | M | n.d. | n.d. | n.d. |
| 119/09 | 51 | M | Binet A | no | Trisomy 12q13 |
| 120/09 | 54 | M | Binet A | no | FISH - no changes |
| 164/09 | 69 | W | Binet A | Leukeran, Alemtuzumab | del17p13, del13q14 and del6q21 |

| | | | | | |
|---|---|---|---|---|---|
| n.d. - not determined | | | | | |

As shown in Figure 6E, ADPGK and GPD2 mRNA levels are significantly up-regulated in CLL cells. This suggests an universal role for the described mechanism of Warburg effect-mediated ROS production/NF-κB activation and indicates its possible importance for tumorigenesis.

### Conclusion

T cell activation is associated with a metabolic shift from mitochondrial respiration towards aerobic glycolysis (*12*). This so-called "Warburg phenotype" is a characteristic feature of fast proliferating normal but also of malignant cells (*11,14*). Previous work has shown, that proliferating T cells are characterized by a high TCR-triggered mitochondrial ROS production (*1, 3*). This mitochondria-generated oxidative signal contributes to NF-κB activation and, thus, stimulates proliferation and an inflammatory response. Interestingly, cancer cells often display an aerobic glycolytic phenotype and are endowed with high intrinsic ROS production and constitutive activation of the NF-κB pathway (*14,16,18,42*). In addition, up-regulation of glycolysis upon hyperglycemic or hypoxic conditions is known to increase mitochondrial ROS generation (*18, 19, 21*), while T cell activation depends on glucose uptake (*9, 10*). Thus, the metabolic analysis of T cell activation-induced mitochondrial ROS production may provide a molecular link connecting these different phenomena at the signaling level.

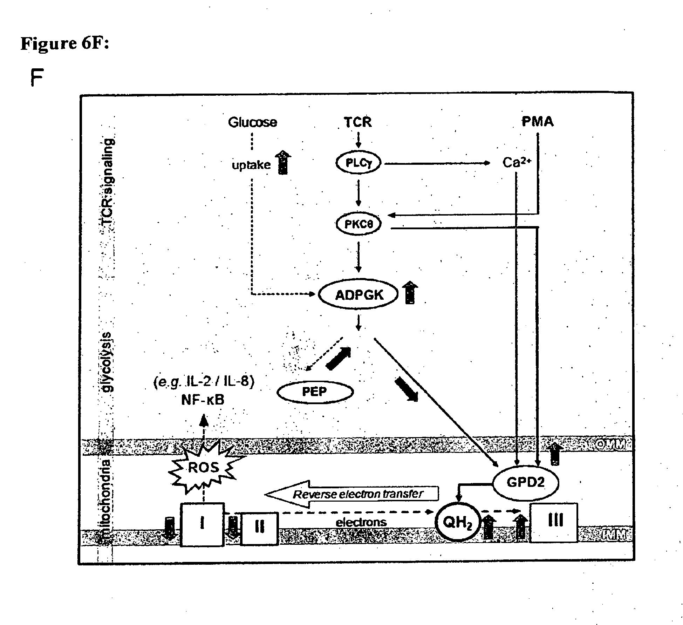

In the experiments described above it was shown that T cell activation-induced mitochondrial ROS production and gene expression depend on triggering of ADPGK, an archaeabacterial protein with so far unknown function in eukaryotes. ADPGK activation is accompanied by a rapid glucose uptake, down-regulation of mitochondrial oxygen consumption and deviation of glycolysis towards GPD shuttle, i.e. the Warburg-effect. In turn, the activation of respiratory chain-associated GPD2 leads to a hyper-reduction of ubiquinone and RET-mediated ROS release from complex I (Figure 6F). This is paralleled by major changes of mitochondrial bioenergetics and ultrastructure. Noteworthy, all events occur within 1 h upon TCR activation. Down-regulation of ADPGK or GPD2 abundance inhibits activation-induced ROS generation and NF-κB-dependent gene expression, whereas over-expression of ADPGK results in their up-regulation.

Strikingly, CLL cells, endowed with a high intrinsic ROS level and constitutive NF-κB activity (*15,17*), display enhanced ADPGK and GPD2 expression (Figure 6E). Furthermore, it is known that glycerol-3-phosphate-mediated respiration and GPD2 activation lead to ROS production (also via RET) (*26, 27, 34*). Thus, the finding of a crucial role for GPD2 in TCR-triggered ROS generation supports a cancer-specific link between increased GPD2 activity/expression and high ROS levels (*35, 36*). In addition, it sheds a new light on the inflammatory phenotype of T cells in diabetic and obese patients (*9, 46*). A re-direction of glycolytic flux in favor of metabolic precursor synthesis by the low-affinity, dimeric form of PK-M2 is a new focus in cancer metabolism (*47*). A recent report describes PEP-mediated PGM activation in fast proliferating and PK-M2 expressing cells resulting in an alternative glycolytic pathway (*29*). Paradoxically, re-directed glycolysis omits the second glycolytic step of ATP generation, an energetic drawback that may be over-come by ADP-mediated glucose phosphorylation. Thus, the described interplay of signaling and metabolic pathways may have a profound meaning for tumorigenesis.

In general, immune cell activation is connected with an inflammatory scenario. The finding of enhanced ADPGK activity at a pro-inflammatory temperature underlines the importance of this novel protein in the NF-κB-mediated response. Moreover, close similarity between predicted secondary structure of human ADPGK and crystal structures of ADPGK proteins of thermophilic *Archaea* (Figure 14) is indicative for thermostability of ADPGK (*40, 41*). A relatively low sequence homology could exemplify convergent evolution of a human ADPGK.

Cellular localization of ADPGK presents an interesting and yet unresolved issue. It has been previously suggested that ADP-mediated glucose phosphorylation constitutes a mechanism to preserve the intracellular ATP pool under ischemic or hypoxic conditions (*38*). ER-localization, however, indicates a more complex role for this protein. ADPGK could participate in metabolic pathways mediated by hexose-6-phosphate dehydrogenase (H6PD) (*48*) or glucose-6-phosphate phosphatase (G6PC3) (*49*), two gluconeogenetic enzymes also expressed highly in extrahepatic tissues. Glucose-6-phosphate/glucose recycling by G6PC3 has been recently demonstrated to be crucial for activation of human neutrophils (*49*). Alternatively, ADPGK-generated glucose-6-phosphate could fuel H6PD-derived NAPDH generation, and, thus influence the ER redox balance. Furthermore, in adipose, liver and muscle tissue H6PD-generated NADPH directly contributes to 11β-hydroxysteroid dehydrogenase type 1 (HSD11B1)-mediated pre-receptorial activation of glucocorticoids (*48*). Thus, ADPGK/H6PD/(HSD11B1) or ADPGK/G6PC3 pathways could participate in regulation of T cell activation and differentiation.

In conclusion, experimental evidence for an unexpected role for ADPGK as a novel regulator of T cell activation is provided. In addition, based on the identified mechanism of glycolytic flux-induced mitochondrial ROS release for T cells, a hypothesis connecting the Warburg phenotype with increased mitochondrial ROS levels and enhanced NF-κB signaling in fast proliferating cancer cells is proposed.

### List of references

1. M. M. Kaminski et al., J Immunol 184, 4827 (May 1, 2010).
2. W. Droge, Physiol Rev 82, 47 (Jan, 2002).
3. M. Kaminski, M. Kiessling, D. Suss, P. H. Krammer, K. Gulow, Mol Cell Biol 27, 3625 (May, 2007).
4. K. Gulow et al., J Immunol 174, 5249 (May 1, 2005).
5. J. S. Yi, B. C. Holbrook, R. D. Michalek, N. G. Laniewski, J. M. Grayson, J Immunol 177, 852 (Jul 15, 2006).
6. S. H. Jackson, S. Devadas, J. Kwon, L. A. Pinto, M. S. Williams, Nat Immunol 5, 818 (Aug, 2004).
7. M. Los et al., Embo J 14, 3731 (Aug 1, 1995).
8. J. Kwon et al., Sci Signal 3, ra59 (Aug 3, 2010).
9. F. B. Stentz, A. E. Kitabchi, Biochem Biophys Res Commun 335, 491 (Sep 23, 2005).
10. S. R. Jacobs et al., J Immunol 180, 4476 (Apr 1, 2008).
11. O. Warburg, Science 124, 269 (Aug 10, 1956).
12. T. Wang, C. Marquardt, J. Foker, Nature 261, 702 (Jun 24, 1976).
13. C. J. Fox, P. S. Hammerman, C. B. Thompson, Nat Rev Immunol 5, 844 (Nov, 2005).
14. M. G. Vander Heiden, L. C. Cantley, C. B. Thompson, Science 324, 1029 (May 22, 2009).
15. Y. Zhou, E. O. Hileman, W. Plunkett, M. J. Keating, P. Huang, Blood 101, 4098 (May 15, 2003).
16. S. I. Grivennikov, M. Karin, Curr Opin Genet Dev 20, 65 (Feb, 2010).
17. J. Tomic, B. Lichty, D. E. Spaner, Blood, (Jan 4, 2011).
18. K. E. Wellen, C. B. Thompson, Mol Cell 40, 323 (Oct 22, 2010).
19. M. Brownlee, Nature 414, 813 (Dec 13, 2001).
20. E. L. Bell et al., J Cell Biol 177, 1029 (Jun 18, 2007).
21. R. B. Hamanaka, N. S. Chandel, Curr Opin Cell Biol 21, 894 (Dec, 2009).
22. M. Bental, C. Deutsch, Magn Reson Med 29, 317 (Mar, 1993).
23. A. Miccheli et al., Biochimie 88, 437 (May, 2006).
24. G. Arismendi-Morillo, Biochim Biophys Acta, (Nov 9).
25. C. A. Mannella, Biochim Biophys Acta 1763, 542 (May-Jun, 2006).
26. A. J. Lambert, M. D. Brand, Methods Mol Biol 554, 165 (2009).
27. S. Miwa, J. St-Pierre, L. Partridge, M. D. Brand, Free Radic Biol Med 35, 938 (Oct 15, 2003).
28. J. Krivanek, L. Novakova, FEBS Lett 282, 32 (Apr 22, 1991).
29. M. G. Vander Heiden et al., Science 329, 1492 (Sep 17, 2010).
30. T. Hitosugi et al., Sci Signal 2, ra73 (2009).
31. T. Hruz et al., Adv Bioinformatics 2008, 420747 (2008).
32. K. Y. Tu et al., Biochem Biophys Res Commun 207, 183 (Feb 6, 1995).
33. R. M. Denton, Biochim Biophys Acta 1787, 1309 (Nov, 2009).
34. L. Tretter, K. Takacs, V. Hegedus, V. Adam-Vizi, J Neurochem 100, 650 (Feb, 2007).
35. S. K. Chowdhury, A. Gemin, G. Singh, Biochem Biophys Res Commun 333, 1139 (Aug 12, 2005).
36. S. K. Chowdhury, S. Raha, M. A. Tarnopolsky, G. Singh, Free Radic Res 41, 1116 (Oct, 2007).
37. J. E. Wilson, J Exp Biol 206, 2049 (Jun, 2003).
38. R. S. Ronimus, H. W. Morgan, Biochem Biophys Res Commun 315, 652 (Mar 12, 2004).
39. C. Wu et al., Genome Biol 10, R130 (2009).
40. V. Guixe, F. Merino, IUBMB Life 61, 753 (Jul, 2009).
41. H. Tsuge et al., Protein Sci 11, 2456 (Oct, 2002).
42. E. O. Hileman, J. Liu, M. Albitar, M. J. Keating, P. Huang, Cancer Chemother Pharmacol 53, 209 (Mar, 2004).
43. A. Gaiger, D. Heintel, U. Jager, Eur J Clin Invest 34 Suppl 2, 25 (Aug, 2004).
44. M. Lopez-Guerra, D. Colomer, Expert Opin Ther Targets 14, 275 (Mar).
45. D. Colomer, J. L. Vives-Corrons, A. Pujades, R. Bartrons, Cancer Res 47, 1859 (Apr 1, 1987).
46. F. B. Stentz, A. E. Kitabchi, Curr Drug Targets 4, 493 (Aug, 2003).
47. S. Mazurek, H. C. Drexler, J. Troppmair, E. Eigenbrodt, U. R. Rapp, Anticancer Res 27, 3963 (Nov-Dec, 2007).
48. G. Banhegyi, M. Csala, A. Benedetti, J Mol Endocrinol 42, 283 (Apr, 2009).
49. H. S. Jun et al., Blood 116, 2783 (Oct 14, 2011).
50. H PELICANO ET AL: "Glycolysis inhibition for anticancer treatment", ONCOGENE, vol. 25, no. 34, 7 August 2006 (2006-08-07), pages 4633-4646, XP055009657
51. SCATENA ROBERTO ET AL: "Glycolytic enzyme inhibitors in cancer treatment.", EXPERT OPINION ON INVESTIGATIONAL DRUGS, vol. 17, no. 10, October 2008 (2008-10), pages 1533-1545, XP002661595
52. N. J. MACIVER ET AL: "Glucose metabolism in lymphocytes is a regulated process with significant effects on immune cell function and survival", JOURNAL OF LEUKOCYTE BIOLOGY, vol. 84, no. 4, 1 October 2008 (2008-10-01), pages 949-957, XP055009842
53. KRAWCZYK CONNIE M ET AL: "Toll-like receptor-induced changes in glycolytic metabolism regulate dendritic cell activation", BLOOD, vol. 115, no. 23, June 2010 (2010-06), pages 4742-4749, XP002661596
54. RICHTER SUSAN ET AL: "Role of the novel glucose-phosphorylating enzyme ADP-dependent glucokinase in human cancer cell lines featuring knockouts generated by zinc finger nucleases", PROCEEDINGS OF THE AMERICAN ASSOCIATION FOR CANCER RESEARCH ANNUAL MEETING, vol. 51 April 2011 (2011-04), XP002661597 & 101 ST ANNUAL MEETING OF THE AMERICAN-ASSOCIATION-FOR-CANCER-RESEARCH; WASHINGTON, DC, USA; APRIL 17 -21, 2010

## Claims

1. A compound capable of reducing or inhibiting (a) the biological activity of ADP-dependent glucokinase (ADPGK) or (b) the expression of the gene encoding ADPGK for use in a method of treating a neoplasm, an autoimmune disease or Graft-versus-Host-Disease (GvHD),
wherein said compound is
- an antisense oligonucleotide or an siRNA reducing or inhibiting the expression of the gene encoding ADPGK,
- an antibody directed against ADPGK or a fragment thereof having the same specificity,
- an inactive version of ADPGK, or
- a polynucleic acid encoding an inactive version of ADPGK.

2. The compound according to claim 1 for the use according to claim 1, wherein the neoplasm to be treated shows ADPGK (over)expression.

3. The compound according to claim 1 for the use according to claim 2, wherein the neoplasm to be treated is B cell chronic lymphocytic leukemia (CLL) or a tumor showing enhanced NF-KB levels.

4. The compound according to claim 1 for the use according to claim 1, wherein said autoimmune disease is rheumatism, Lupus erythematodes, Psoriasis, atopic dermatitis, multiple sclerosis, or diabetes.

5. An in-vitro method for identifying an anti-cancer compound or an immune suppressive modulator compound, said compound being capable of modulating the biological activity of ADPGK or the expression of the gene encoding ADPGK, comprising the steps of:
(a) incubating a candidate compound with a test system comprising ADPGK or the gene encoding ADPGK ; and
(b) assaying a biological activity of ADPGK ;
wherein an increase or reduction of the biological activity of ADPGK is indicative of the presence of a candidate compound having the desired property.

6. The in-vitro method of claim 5, wherein the increase or reduction of the biological activity of ADPGK is determined by comparison to a test system **characterized by** the absence of said test compound.

## Patentansprüche

1. Verbindung, mit der (a) die biologische Aktivität der ADP-abhängigen Glucokinase (ADPGK) oder (b) die Expression des für ADPGK kodierenden Gens reduziert oder gehemmt werden kann, zur Verwendung in einem Verfahren zum Behandeln eines Neoplasmas, einer Autoimmunerkrankung oder einer Transplantat-gegen-Empfänger-Reaktion (Graft-versus-Host-Reaktion, GvHD),
wobei die Verbindung:
- ein Antisense-Oligonukleotid oder eine siRNA, die die Expression des für ADPGK kodierenden Gens reduziert oder hemmt,
- ein Antikörper, der gegen ADPGK oder ein Fragment davon mit derselben Spezifität gerichtet ist,
- eine inaktive Version von ADPGK oder
- eine Polynukleinsäure, die für eine inaktive Version von ADPGK kodiert,
ist.

2. Verbindung nach Anspruch 1, zur Verwendung nach Anspruch 1, wobei das zu behandelnde Neoplasma eine ADPGK (Über)expression zeigt.

3. Verbindung nach Anspruch 1 zur Verwendung nach Anspruch 2, wobei das zu behandelnde Neoplasma eine chronische lymphozytische Leukämie (CLL) vom B-Zelltyp oder ein Tumor mit erhöhten NF-κB-Niveaus ist.

4. Verbindung nach Anspruch 1 zur Verwendung nach Anspruch 1, wobei die Autoimmunerkrankung Rheuma, Lupus erythematodes, Psoriasis, atopische Dermatitis, Multiple Sklerose oder Diabetes ist.

5. In-vitro-Verfahren zum Identifizieren einer Antikrebsverbindung oder einer immunsuppressiven Modulatorverbindung, wobei die Verbindung die biologische Aktivität von ADPGK oder die Expression des für ADPGK kodierenden Gens modulieren kann, umfassend die folgenden Schritte:
(a) Inkubieren einer Kandidatenverbindung mit einem Testsystem, umfassend ADPGK oder das für ADPGK kodierende Gen; und
(b) Untersuchen der biologischen Aktivität von ADPGK;
wobei der Anstieg oder die Verringerung der biologischen Aktivität von ADPGK für das Vorhandensein einer Kandidatenverbindung mit der gewünschten Eigenschaft typisch ist.

6. In-vitro-Verfahren nach Anspruch 5, wobei der Anstieg oder die Verringerung der biologischen Aktivität von ADPGK durch Vergleich mit einem Testsystem bestimmt wird, **gekennzeichnet durch** das Fehlen der Testverbindung.

## Revendications

1. Composé à même de diminuer ou d'inhiber (a) l'activité biologique de la glucokinase dépendant de l'ADP (ADPGK) ou (b) l'expression du gène codant l'ADPGK, destiné à être utilisé dans un procédé de traitement d'un néoplasme, d'une maladie auto-immune ou d'une maladie de greffon contre l'hôte (GvHD),
dans lequel ledit composé est
- un oligonucléotide antisens ou un siARN réduisant ou inhibant l'expression du gène codant l'ADPGK,
- un anticorps dirigé contre l'ADPGK ou un fragment de celui-ci présentant la même spécificité,
- une version inactive de l'ADPGK, ou
- un acide polynucléique codant une version inactive de l'ADPGK.

2. Composé selon la revendication 1 destiné à être utilisé selon la revendication 1, dans lequel le néoplasme à traiter présente une (sur)expression d'ADPGK.

3. Composé selon la revendication 1 destiné à être utilisé selon la revendication 2, dans lequel le néoplasme à traiter est la leucémie lymphoïde chronique (LLC) à cellules B ou une tumeur présentant des niveaux de NF-KB améliorés.

4. Composé selon la revendication 1 destiné à être utilisé selon la revendication 1, dans lequel ladite maladie auto-immune est le rhumatisme, le lupus érythémateux, le psoriasis, la dermatite atopique, la sclérose en plaques ou le diabète.

5. Procédé in vitro d'identification d'un composé anticancéreux ou un composé modulateur immunosuppresseur, ledit composé étant à même de moduler l'activité biologique de l'ADPGK ou l'expression du gène codant l'ADPGK, comprenant les étapes consistant à:
(a) incuber un composé candidat avec un système de test comprenant l'ADPGK ou le gène codant l'ADPGK; et
(b) tester une activité biologique d'ADPGK;
où une augmentation ou une réduction de l'activité biologique d'ADPGK indique la présence d'un composé candidat présentant la propriété souhaitée.

6. Procédé in vitro selon la revendication 5, dans lequel l'augmentation ou la réduction de l'activité biologique d'ADPGK est déterminée par comparaison avec un système de test **caractérisé par** l'absence dudit composé d'essai.
